(19) Europäisches Patentamt
European Patent Office
Office européen des brevets

(11) Veröffentlichungsnummer: **0 306 871 B1**

(12) # EUROPÄISCHE PATENTSCHRIFT

(45) Veröffentlichungstag der Patentschrift: **08.12.93**

(51) Int. Cl.5: **C07D 471/08**, A61K 31/435, //(C07D471/08,221:00,221:00)

(21) Anmeldenummer: **88114428.1**

(22) Anmeldetag: **03.09.88**

Die Akte enthält technische Angaben, die nach dem Eingang der Anmeldung eingereicht wurden und die nicht in dieser Patentschrift enthalten sind.

(54) **3,7-Diazabicyclo [3,3,1] nonan-Verbindungen sowie Verfahren zu ihrer Herstellung und diese Verbindungen enthaltende Arzneimittel.**

(30) Priorität: **09.09.87 DE 3730222**
**09.09.87 DE 3730224**

(43) Veröffentlichungstag der Anmeldung:
**15.03.89 Patentblatt 89/11**

(45) Bekanntmachung des Hinweises auf die Patenterteilung:
**08.12.93 Patentblatt 93/49**

(84) Benannte Vertragsstaaten:
**BE CH DE ES FR GB IT LI NL**

(56) Entgegenhaltungen:
**EP-A- 0 000 074**
**EP-A- 0 103 833**
**DE-A- 2 428 792**

(73) Patentinhaber: **Kali-Chemie Pharma GmbH**
**Hans-Böckler-Allee 20**
**D-30173 Hannover(DE)**

(72) Erfinder: **Schön, Uwe**
**Föhrenkamp 12**
**D-3167 Burgdorf(DE)**
Erfinder: **Kehrbach, Wolfgang**
**Altenbekener Damm 41**
**D-3000 Hannover 1(DE)**
Erfinder: **Buschmann, Gerd**
**Ernst-Ebeling-Strasse 9**
**D-3000 Hannover 72(DE)**
Erfinder: **Kühl, Ulrich Gottfried**
**Franzburger Strasse 10**
**D-3007 Gehrden 1(DE)**
Erfinder: **Ziegler, Dieter**
**Robert-Koch-Strasse 11**
**D-3003 Ronnenberg 1(DE)**

(74) Vertreter: **Lauer, Dieter, Dr.**
**c/o Kali-Chemie Aktiengesellschaft,**
**Hans-Bockler-Allee 20**
**D-30173 Hannover (DE)**

EP 0 306 871 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue 3-Cinnamyl-3,7-diazabicyclo[3,3,1]nonan-Verbindungen und deren Salze sowie diese Verbindungen enthaltende pharmazeutische Zubereitungen und Verfahren und Zwischenprodukte zur Herstellung dieser Verbindungen.

Aus der DE-OS 26 58 558 sind 3-Alkanoyl- und 3-Aroyl-3,7-diazabicyclo[3,3,1]nonan-Derivate bekannt, für welche zentralanalgetische Wirkungen angegeben werden. Aus DE-OS 24 28 792 sind in 3- und 7-Stellung durch Alkyl- oder Phenylalkylreste substituierte 3,7-Diazabicyclo[3,3,1]nonan-Derivate mit antiarrhythmischen Eigenschaften bekannt. In der EP-A-0 000 074 werden 7-Benzyl-3-phenylalkyl-3,7-diazabicyclo[3,3,1]nonan-Derivate mit ebenfalls antiarrhythmischen Wirkungen beschrieben. Weitere 3,7-Diazabicyclo[3,3,1]nonan-Derivate mit antiarrhythmischen Eigenschaften sind aus der EP-A-0 103 833 bekannt.

Der vorliegenden Erfindung liegt die Aufgabe zugrunde, neue 3,7-Diazabicyclo[3,3,1]nonan-Verbindungen mit wertvollen pharmakologischen Eigenschaften zu entwickeln.

Es wurde nun gefunden, daß die neuen in 3-Stellung durch oder einen Cinnamylrest substituierten 3,7-Diazabicyclo[3,3,1]nonan-Verbindungen wertvolle pharmakologische Eigenschaften, insbesondere wertvolle herzwirksame Eigenschaften, besitzen. Sie zeichnen sich durch ein günstiges Wirkungsprofil mit herzfrequenzsenkenden Wirkungen und antiarrhythmischen Eigenschaften aus.

Die vorliegende Erfindung betrifft daher neue 3,7-Diazabicyclo[3,3,1]nonan-Verbindungen der allgemeinen Formel I

(s. Formel I)

worin

- $R^1$ eine Alkylgruppe mit 1-6 Kohlenstoffatomen, eine Cycloalkylalkylgruppe mit 4-9 Kohlenstoffatomen oder Benzyl bedeutet,
- $R^2$ Alkyl mit 1-4 Kohlenstoffatomen bedeutet und
- $R^3$ Alkyl mit 1-4 Kohlenstoffatomen bedeutet oder
- $R^2$ und $R^3$ gemeinsam eine Alkylenkette mit 3-6 Kohlenstoffatomen bilden, und
- R4 eine Cinnamylgruppe der allgemeinen Formel a

(s. Formel a)

bedeutet, worin

- $R^9$ Wasserstoff, Halogen, Alkyl mit 1-4 Kohlenstoffatomen, Alkoxy mit 1-4 Kohlenstoffatomen oder Hydroxy bedeutet,
- $R^{10}$ Wasserstoff, Halogen, Alkoxy mit 1-4 Kohlenstoffatomen, Alkyl mit 1-4 Kohlenstoffatomen, Hydroxy oder, falls $R^9$ Wasserstoff ist, auch Trifluormethyl oder Nitro bedeutet, und
- $R^{11}$ Wasserstoff oder, falls $R^9$ und $R^{10}$ Alkoxy mit 1-4 Kohlenstoffatomen sind, auch Alkoxy mit 1-4 Kohlenstoffatomen bedeutet, und deren Säureadditionssalze.

Sofern in den Verbindungen der Formel I $R^1$ für eine Alkylgruppe steht, kann diese geradkettig oder verzweigt sein und 1 bis 6, vorzugsweise 2 bis 4 Kohlenstoffatome enthalten. Eine Cycloalkylalkylgruppe $R^1$ kann 4 bis 9, vorzugsweise 4 bis 7, Kohlenstoffatome enthalten. Als besonders geeignete Reste $R^1$ haben sich Alkyl- und Cycloalkylalkylreste, insbesondere verzweigte Alkylreste, erwiesen.

Sofern die Substituenten $R^2$ und $R^3$ niederes Alkyl darstellen, können diese Alkylgruppen geradkettig oder verzweigt sein und 1 bis 4, vorzugsweise 1 bis 3 Kohlenstoffatome enthalten. Die Alkylgruppen $R^2$ und $R^3$ sind zweckmäßigerweise gleichartig, können jedoch auch verschieden sein. Sofern $R^2$ und $R^3$ gemeinsam eine Alkylenkette bilden, kann diese 3 bis 6, vorzugsweise 4 bis 5 Kohlenstoffatome enthalten.

In den Verbindungen der Formel I stellt der Rest $R^4$ eine gegebenenfalls substituierte Cinnamylgruppe b dar. Sofern die Substituenten $R^9$ bis $R^{11}$ der Cinnamylgruppe b niedere Alkylgruppen darstellen oder enthalten, können diese 1 bis 4, insbesondere 1 oder 2 Kohlenstoffatome enthalten. Halogensubstituenten $R^9$ und/oder $R^{10}$ stellen vorzugsweise Chlor dar. Vorzugsweise ist der Cinnamylrest $R^4$ unsubstituiert oder durch Halogen oder Methoxy mono- oder auch disubstituiert.

Erfindungsgemäß werden die neuen 3,7-Diazabicyclo[3,3,1]nonan-Verbindungen der allgemeinen Formel I und deren Säureadditionssalze erhalten, indem man in an sich bekannter Weise

a) Verbindungen der allgemeinen Formel II

(s. Formel II)

worin $R^1$, $R^2$ und $R^3$ obige Bedeutung besitzen, mit Verbindungen der allgemeinen Formel III

$R^4$-X III

worin $R^4$ die für $R^4$ oben angegebene Bedeutung besitzt, wobei jedoch allfällige freie Hydroxygruppen

mit einer Schutzgruppe versehen sind, und X eine aminolytisch abspaltbare Gruppe bedeutet, umsetzt oder

Verbindungen der allgemeinen Formel XIV

(s. Formel XIV)

worin $R^1$, $R^2$, $R^3$ und $R^{11}$ obige Bedeutung besitzen und $R^9$ und $R^{10}$ die oben für $R^9$ und $R^{10}$ angebenene Bedeutung besitzen, wobei jedoch allfällige freie Hydroxygruppen mit einer Schutzgruppe versehen sind, und Z und Z' gemeinsam Sauerstoff bedeuten oder Z für Hydroxy und Z' für Wasserstoff stehen, reduziert,

und anschließend allfällige Hydroxyschutzgruppen wieder abspaltet, und gewünschtenfalls freie Verbindungen der Formel I in ihre Säureadditionssalze überführt oder die Säureadditionssalze in die freien Verbindungen der Formel I überführt.

Die Umsetzung von Verbindungen der Formel II mit Verbindungen der Formel III kann auf an sich bekannte Weise unter zur Alkylierung von Aminen üblichen Bedingungen erfolgen. So wird die Umsetzung zweckmäßigerweise in einem unter den Reaktionsbedingungen inerten organischen Lösungsmittel unter basischen Bedingungen durchgeführt. Als aminolytisch abspaltbare Reste in den Verbindungen der Formel III kommen bevorzugt Halogene wie Chlor oder Brom oder auch organische Sulfonsäurereste in Frage, beispielsweise Rest von Niederalkansulfonsäuren wie z.B. Methansulfonsäure oder von aromatischen Sulfonsäuren wie Benzolsulfonsäure oder durch niederes Alkyl oder Halogen substituierten Benzolsulfonsäuren, z.B. Toluolsulfonsäuren oder Brombenzolsulfonsäuren. Als inerte organische Lösungsmittel eignen sich insbesondere aprotische Lösungsmittel wie beispielsweise Äther, insbesondere cyclische Äther wie Tetrahydrofuran, Dimethylformamid, aromatische Kohlenwasserstoffe wie Benzol oder Toluol, oder Gemische aus den vorgenannten Lösungsmitteln. Zweckmäßigerweise wird die Reaktion in Gegenwart mindestens einer äquivalenten Menge einer Base durchgeführt. Beispiele geeigneter Basen sind Alkalimetallcarbonate, Alkalimetallamide, Alkalimetallhydride, lithiumorganische Verbindungen wie Niederalkyllithium oder Phenyllithium. So erweist sich beispielsweise die Verwendung von Kaliumcarbonat in Dimethylformamid oder von n-Butyllithium in Tetrahydrofuran oder von Lithiumamid in Tetrahydrofuran oder Dimethylformamid als zweckmäßig. Die Reaktionstemperatur kann je nach Art der verwendeten Base variieren und zwischen ca. 0 °C und Siedetemperatur des Lösungsmittels, insbesondere zwischen ca. 0 °C und 80 °C gewählt werden. Die Reaktionsdauer kann je Art der gewählten Reaktionsbedingungen zwischen 2 und 12 Stunden betragen.

Falls der Rest $R^4$ der Verbindungen der Formel III freie Hydroxysubstituenten enthält, müssen diese während der Umsetzung mit den Verbindungen der Formel II auf an sich bekannte Weise durch leicht wieder abspaltbare Schutzgruppen geschützt werden. Geeignete nach der Reaktion leicht wieder abspaltbare Schutzgruppen für phenolische OH-Gruppen sind z.B. bekannt aus E. McOmie "Protective Groups in Organic Chemistry" Plenum Press 1971. Beispielsweise eignen sich zum Schutz einer Hydroxylgruppe Ester, z.B. Acetate und leicht abspaltbare Äther, insbesondere Tetrahydropyranyläther oder leicht abspaltbare Carbonate wie Benzylcarbonate. Es müssen solche Schutzgruppen gewählt werden, welche anschließend leicht unter Bedingungen, unter denen die Doppelbindung des Cinnamylrestes nicht angegriffen wird, abspaltbar sind.

Die Reduktion von Verbindungen der Formel XIV gemäß Verfahrensvariante b kann nach an sich zur Reduktion von Amiden und Aminocarbinolen üblichen Methoden erfolgen. Als Reduktionsmittel eignen sich komplexe Metallhydride. So erweisen sich beispielsweise zur Reduktion von Amiden der Formel XIV komplexe Aluminiumhydride, wie Lithiumaluminiumhydrid oder Natrium-bis(2-methoxyäthoxy)-dihydroaluminat in einem unter den Reaktionsbedingungen inerten Lösungsmittel, beispielsweise einem offenkettigen oder cyclischen Äther wie Diäthyläther oder Tetrahydrofuran, gegebenenfalls im Gemisch mit aromatischen Kohlenwasserstoffen wie Benzol oder Toluol als geeignet. Zur Reduktion von Aminocarbinolen der Formel XIV eignet sich außerdem auch Natriumborhydrid. Die Reduktion mit Natriumborhydrid kann in einem niederen Alkohol, beispielsweise Methanol, gegebenenfalls im Gemisch mit anderen inerten organischen Lösungsmitteln erfolgen. Die Reaktionstemperatur kann je nach Art des verwendeten Reduktionsmittels variieren. Als günstig erweisen sich Temperaturen zwischen 0 °C und Raumtemperatur.

Die Verbindungen der Formel I können auf an sich bekannte Weise aus dem Reaktionsgemisch isoliert und gereinigt werden. Säureadditionssalze können in üblicher Weise in die freien Basen überführt werden und diese gewünschtenfalls in bekannter Weise in pharmakologisch verträgliche Säureadditionssalze überführt werden. Als pharmakologisch annehmbare Säureadditionssalze der Verbindungen der Formel I eignen sich beispielsweise deren Salze mit anorganischen Säuren, z.B. Halogenwasserstoffsäuren, insbesondere Chlorwasserstoffsäure, Schwefelsäure oder Phosphorsäuren, oder mit organischen Säuren, beispielsweise niederen aliphatischen Mono- oder Dicarbonsäuren wie Milchsäure, Maleinsäure, Fumarsäure, Weinsäure oder Essigsäure, oder Sulfonsäuren, beispielsweise Niederalkylsulfonsäuren wie Methansulfonsäure oder gegebenenfalls im Benzolring durch Halogen oder niederes Alkyl substituierte Benzolsulfonsäuren wie p-Toluolsulfonsäure oder Cyclohexylaminosulfonsäure.

Für den Fall, daß $R^2$ und $R^3$ verschieden sind, können die Verbindungen in zwei stereoisomeren Formen auftreten. Die vorliegende Erfindung umfaßt sowohl die Isomerengemische wie auch die reinen Isomeren dieser Verbindungen der Formel I. Isomerengemische können auf an sich bekannte Weise auf der Stufe der Endverbindungen oder auf einer Zwischenproduktstufe in die einzelnen Isomeren aufgetrennt werden, beispielsweise durch fraktionierte Kristallisation oder durch säulenchromatographische Trennung.

Der substituierte Cinnamylrest $R^4$ kann cis- oder trans-Konfiguration haben.

Die als Ausgangsverbindungen verwendeten 3,7-Diazabicyclo[3,3,1]nonan-Verbindungen der Formel II sind aus der EP-A-0 103 833 und der DE-OS 26 58 558 bekannt und/oder können nach den in diesen Schriften beschriebenen Methoden oder analog zu den in diesen Schriften beschriebenen Methoden auf an sich bekannte Weise hergestellt werden. Beispielsweise können Verbindungen der Formel II erhalten werden, indem man aus Verbindungen der Formel IV

(s. Formel IV)

worin $R^1$, $R^2$ und $R^3$ obige Bedeutung besitzen und $R^{12}$ Benzyl bedeutet, die Benzylgruppe $R^{12}$ auf an sich bekannte Weise hydrogenolytisch abspaltet. Die hydrogenolytische Abspaltung der Gruppe $R^{12}$ kann mit Wasserstoff in Gegenwart eines Palladium/Kohle-Katalysators in einem organischen protischen, polaren Lösungsmittel, beispielsweise einem niederen Alkohol wie Äthanol, zweckmäßigerweise in Gegenwart katalytischer Mengen Eisessig erfolgen. Die Hydrierung kann zweckmäßig bei Raumtemperatur und einem Wasserstoffdruck von ca. 5 bis 6 Atmosphären durchgeführt werden.

Ausgangsverbindungen der Formel IV können beispielsweise ausgehend von Verbindungen der Formel V

(s. Formel V)

worin $R^1$, $R^2$ und $R^3$ obige Bedeutung besitzen, erhalten werden. Hierzu werden die Tetraoxo-Verbindungen der Formel V zunächst mit Benzylhalogeniden der Formel VI

(s. Formel VI)

worin $R^{12}$ obige Bedeutung besitzt und Hal für Halogen, insbesondere Chlor oder Brom, steht, zu den N,N'-disubstituierten Tetraoxo-Verbindungen der Formel VII

(s. Formel VII)

worin $R^1$, $R^2$, $R^3$ und $R^{12}$ obige Bedeutung besitzen, umgesetzt und diese anschließend zu den Verbindungen der Formel IV reduziert. Die Umsetzung der Diimid-Verbindungen der Formel V mit den Verbindungen der Formel VI kann nach an sich zur Alkylierung von Imiden üblichen Methoden erfolgen.

Die Umsetzung findet zweckmäßigerweise in einem unter den Reaktionsbedingungen inerten Lösungsmittel in Gegenwart einer Base bei erhöhter Temperatur, beispielsweise Siedetemperatur des Lösungsmittels, statt. So eignen sich beispielsweise Alkalimetallcarbonate, -amide oder -hydride in Dimethylformamid oder Alkalimetallalkoholate in einem niederen Alkohol. Zweckmäßigerweise wird das Benzylhalogenid im Überschuß eingesetzt.

Die 2,4,6,8-Tetraoxo-3,7-diazabicyclo[3,3,1]nonan-Verbindungen der Formel V sind bekannt und/oder können nach der von Hörlein beschriebenen Methode (Eur. J. Med. Chem. 12, 301-305) hergestellt werden durch Ringschluß von 2,6-Dioxo-3,5-dicyanpiperidin-Verbindungen der Formel VIII

(s. Formel VIII)

worin $R^1$, $R^2$ und $R^3$ obige Bedeutung besitzen, in hochprozentigen Säure-Wasser-Gemischen. Die 2,6-Dioxo-3,5-dicyanpiperidine VIII werden ihrerseits auf bekannte Weise erhalten durch Kondensation von entsprechend substituierten Alkylidencyanessigestern der Formel IX

(s. Formel IX)

worin $R^2$ und $R^3$ obige Bedeutung besitzen, mit Cyanacetamiden der Formel X

(s. Formel X)

worin $R^1$ obige Bedeutung besitzt.

Verbindungen der Formel IIa

(s. Formel IIa)

worin $R^2$ und $R^3$ obige Bedeutung besitzen und $R^{1'}$ die für $R^1$ angegebene Bedeutung mit Ausnahme von Benzyl besitzt, können auch erhalten werden, indem man Verbindungen der Formel II, worin $R^1$ Benzyl bedeutet, mit Verbindungen der Formel XI

(s. Formel XI)

worin $R^{1'}$ und X obige Bedeutung besitzen, alkyliert und anschließend die Benzylgruppe hydrogenolytisch abspaltet. Die Alkylierung erfolgt auf an sich bekannte Weise z.B. unter den für die Umsetzung der Verbindungen der Formel II mit Verbindungen der Formel III angegebenen Bedingungen.

Verbindungen der Formel XIV'

(s. Formel XIV')

worin $R^1$, $R^2$, $R^3$, $R^9$, $R^{10}$, Z und Z' die oben für Formel XIV angegebene Bedeutung besitzen, und $R^{11}$ Wasserstoff oder, falls $R^9$ und $R^{10}$ Alkoxy mit 1-4 Kohlenstoffatomen sind und $R^2$ und $R^3$ unabhängig voneinander Alkyl mit 1-4 Kohlenstoffatomen sind, auch Alkoxy mit 1-4 Kohlenstoffatomen bedeutet, sind in der Litaratur bisher noch nicht beschrieben worden. Erfindungsgemäß stellen die Verbindungen der Formel XIV wertvolle Zwischenprodukte zur Herstellung von pharmakologisch aktiven Verbindungen, beispielsweise Verbindungen der Formel I dar.

Die Amide der Formel XIVa

(s. Formel XIVa)

worin $R^1$, $R^2$, $R^3$, $R^9$, $R^{10}$ und $R^{11}$ obige Bedeutung besitzen, können auf an sich bekannte Weise erhalten werden, indem man Säuren der Formel XV

(s. Formel XV)

worin $R^9$, $R^{10}$ und $R^{11}$ obige Bedeutung besitzen oder deren reaktive Säurederivate mit Verbindungen der Formel II umsetzt. Die Umsetzung von Säuren der Formel XV und ihren reaktiven Derivaten mit Verbindungen der Formel II kann nach an sich zur Amidbildung durch Aminoacylierung üblichen Methoden durchgeführt werden. Zweckmäßig werden die Säuren in an sich bekannter Weise durch Überführung in ein reaktionsfähiges Derivat aktiviert. Als reaktionsfähige Säurederivate kommen beispielsweise Säurehalogenide, insbesondere Chloride oder Bromide, niedere Alkylester oder gemischte Anhydride, z.B. Anhydride mit niederen Alkancarbonsäuren oder niederen Alkansulfonsäuren, insbesondere Essigsäure oder Methansulfonsäure in Frage. So eignen sich zur Umsetzung mit den Verbindungen der Formel II Verbindungen der Formel XVa

(s. Formel XVa)

worin $R^9$, $R^{10}$ und $R^{11}$ obige Bedeutung besitzen und Y Hydroxy, niederes Alkoxy, Halogen oder eine Acyloxygruppe -OY' bedeutet, worin Y' für niederes Alkylcarbonyl oder niederes Alkylsulfonyl steht.

Die Überführung der freien Säuren der Formel XV in reaktive Säurederivate erfolgt auf an sich bekannte Weise. So können Säurehalogenide der Formel XVa z.B. durch Umsetzung der Säuren der Formel XV mit einem Halogenierungsmittel, beispielsweise Phosphortrichlorid, Phosphorpentabromid oder Thionylchlorid erhalten werden. Gewünschtenfalls kann die Umsetzung in Gegenwart von Pyridin oder einer anderen tertiären organischen Base durchgeführt werden. Gemischte Säureanhydride können z.B. durch Umsetzung von Säuren der Formel XV oder deren Alkalimetallsalzen mit einem entsprechenden organischen Säurechlorid in einem unter den Reaktionsbedingungen inerten organischen Lösungsmittel, beispielsweise einem Halogenkohlenwasserstoff, gegebenenfalls in Gegenwart einer tertiären organischen Base, beispielsweise Pyridin erhalten werden.

Die Umsetzung der Säurederivate der Formel XVa mit den Verbindungen der Formel II kann in einem unter den Reaktionsbedingungen inerten Lösungsmittel bei Temperaturen zwischen -30 °C und Siedetemperatur des Lösungsmittels erfolgen. Als Lösungsmittel eignen sich halogenierte Kohlenwasserstoffe wie Dichlormethan oder Chloroform, aromatische Kohlenwasserstoffe wie Benzol oder Toluol, cyclische Äther wie Tetrahydrofuran oder Dioxan oder Gemische dieser Lösungsmittel. Gewünschtenfalls kann die Umsetzung in Gegenwart eines säurebindenden Reagenzes durchgeführt werden. Als säurebindende Mittel eignen sich anorganische Basen, insbesondere Alkalimetallcarbonate und organische Basen, insbesondere tertiäre Niederalkylamine und Pyridine.

Aminocarbinolverbindungen der Formel XIVb

(s. Formel XIVb)

worin $R^1$, $R^2$, $R^3$, $R^9$, $R^{10}$ und $R^{11}$ obige Bedeutung besitzen, bilden sich beispielsweise, wenn man Verbindungen der Formel II mit einem Aldehyd der Formel XVI

(s. Formel XVI)

worin $R^9$, $R^{10}$ und $R^{11}$ obige Bedeutung besitzen, kondensiert, und werden zweckmäßigerweise direkt in situ gemäß Verfahrensvariante b zu Verbindungen der Formel Ia weiter reduziert.

Die Umsetzung der Aldehyde der Formel XVI mit den Verbindungen der Formel II kann nach an sich zur Herstellung von Aminoalkoholen üblichen Methoden durchgeführt werden. Beispielsweise kann die Kondensation der Aldehydverbindungen der Formel XVI mit den cyclischen Aminverbindungen der Formel II durch Erhitzen in einem unter den Reaktionsbedingungen inerten polaren Lösungsmittel, beispielsweise einem niederen Alkohol wie Methanol, erfolgen.

Die Säuren der Formel XV und die Aldehyde der Formel XVI sind bekannt und/oder können auf an sich bekannte Weise hergestellt werden.

Die Verbindungen der Formel III sind bekannt und/oder können auf an sich bekannte Weise erhalten werden.

Verbindungen der Formel III, können beispielsweise ausgehend von Säuren der Formel XV oder deren Niederalkylestern oder Aldehyden der Formel XVI erhalten werden, indem man diese zunächst zu Carbinolverbindungen der Formel XVII

(s. Formel XVII)

worin $R^9$, $R^{10}$ und $R^{11}$ obige Bedeutung besitzen, reduziert und anschließend in an sich bekannter Weise die alkoholische Hydroxygruppe in eine aminolytisch abspaltbare Gruppe X überführt. Als Reduktionsmittel zur Reduktion der Säuren der Formel XV oder deren niederen Alkylestern und der Aldehyde der Formel XVI zu den Carbinolen der Formel XVII eignen sich komplexe Metallhydride, beispielsweise Lithiumaluminiumhydride und im Falle von Aldehyden auch Natriumborhydrid. Zur Einführung eines Halogenidrestes X können die Carbinol-Verbindungen der Formel XVII beispielsweise mit der entsprechenden Halogenwasserstoffsäure umgesetzt werden. Hierzu wird die Verbindung der Formel XVII zweckmäßigerweise in einer wäßrigen Lösung der Halogenwasserstoffsäure zum Sieden erhitzt. Die Einführung eines Chlorid kann auch in an sich bekannter Weise durch Umsetzen mit Thionylchlorid erfolgen. Zur Einführung eines Sulfonsäurerestes X werden die Verbindungen der Formel XVII zweckmäßigerweise mit dem entsprechenden Sulfonsäurechlorid umgesetzt. Die Umsetzung kann beispielsweise in einem inerten Lösungsmittel, z.B. einem cyclischen Äther wie Tetrahydrofuran oder einem halogenierten Kohlenwasserstoff wie Dichlormethan bei Raumtemperatur erfolgen.

Die erfindungsgemäßen neuen Verbindungen der Formel I und ihre pharmakologisch akzeptablen Säureadditionssalze besitzen interessante pharmakologische Eigenschaften, insbesondere herzkreislaufwirksame Eigenschaften. Die Verbindungen zeichnen sich durch ausgeprägte herzfrequenzsenken Wirkungen mit einem günstigen Wirkungsprofil aus. So weisen die Verbindungen neben bradykarden Wirkungen auch antiarrhythmische Eigenschaften auf, ohne den Sauerstoffbedarf des Herzens und den Blutdruck ungünstig zu beeinflussen.

Die herzwirksamen Eigenschaften der Verbindungen lassen sich in pharmakologischen Standardtestmethoden in vitro und in vivo nachweisen.

I. In-vitro-Nachweis der herzfrequenzsenkenden Wirkung und der antiarrhythmischen Wirkung.

Der direkte Einfluß der Wirksubstanzen auf die Herzfrequenz (FRQ) wurde an spontan schlagenden, isolierten rechten Vorhöfen von männlichen Pirbright-white Meerschweinchen der Gewichtsklasse 250-300 g geprüft. In der nachfolgenden Tabelle I ist als FRQ 75 diejenige Konzentration in µmol/l angegeben, bei der es 20 Minuten nach der Substanzgabe zu einer Abnahme der Frequenz auf 75 % des Ausgangswertes kommt.

Der Nachweis der antiarrhythmischen Wirkungen der Testsubstanzen erfolgte experimentell durch Bestimmung der funktionellen Refraktärzeit (= FRP) des linken elektrisch gereizten (1 Hz) Herzvorhofes von männlichen Pirbrightwhite Meerschweinchen der Gewichtsklasse 250-300 g mit Hilfe der gepaarten elektrischen Stimulation in Anlehnung an die Methode von Govier (W.C. Govier, J. Pharmacol. Exp. Ther. 148 (1) (1965), 100 bis 105). In der nachfolgenden Tabelle I ist als FRP +25 ms diejenige Konzentration in µmol/l angegeben, bei der es 18 Minuten nach der Substanzapplikation zu einer Verlängerung der funktionellen Refraktärzeit um 25 ms kommt.

Die für die Testsubstanzen in der Tabelle I angegebenen Beispielsnummern beziehen sich auf die nachstehenden Herstellungsbeispiele.

Tabelle I

| Testsubstanz der Formel I Substanz Beispiel Nr. | Herzwirksame Eigenschaften Effektive Konzentration in µmol/l zur Erreichung von | |
|---|---|---|
| | FRQ 75 % | FRP +25 ms |
| 8 | 0,74 | 4,3 |
| 5 | 0,89 | 7,9 |
| 9 | 1,2 | 8,9 |

II. Untersuchungen in vivo an der narkotisierten Ratte.

Die Wirkung der Substanzen auf Herzfrequenz und Blutdruck bei i.v.-Dauerinfusion in narkotisierten Ratten wurde nach der Methode von Buschmann et al. (J. Cardiovascular Pharmacol. 2, 777-781 (1980)) bestimmt.

Männliche Wistar-Ratten (330-370 g Körpergewicht) wurden durch i.p.-Applikation von 1,25 g/kg Urethan narkotisiert und tracheotomiert. Nach einer Äquilibrierungsphase von 10 Minuten wird mit den Messungen begonnen. In einer Vorlaufphase von 5 Minuten werden die Ausgangswerte gemessen. Anschließend werden die Testsubstanzen gelöst in isotonischer Natriumchloridlösung (ggf. mit Zusatz eines Lösungsvermittlers) als Dauerinfusion i.v. appliziert, beginnend mit der niedrigsten Dosis. Die Dosis wird alle 10 Minuten ohne Erhöhung des Infusionsvolumens auf das 10-fache gesteigert. Es werden der systolische und der diastolische Blutdruck (Ps und Pd) gemessen und daraus der mittlere Blutdruck (Pm) bestimmt. Gleichzeitig wird aus dem Elektrocardiogramm (EKG) die Herzfrequenz aus dem R-R-Abstand bestimmt. In der nachfolgenden Tabelle II werden für jede Testtiergruppe die gemessenen Ausgangswerte (= Vorwerte) für die Herzfrequenz (FRQ) und den diastolischen Blutdruck (Pd) sowie die bei einer Testsubstanzdosis von 10 µmol/kg gemessenen Werte angegeben und die Änderung dieser Parameter in % berechnet.

Tabelle II

| Einfluß auf Herzfrequenz (FRQ) und diastolischen Blutdruck (Pd) | | | | | |
|---|---|---|---|---|---|
| Testsubstanz Beispiel Nr. | Dosis µmol/kg | FRQ $min^{-1}$ | % Änderung FRQ | Pd mm Hg | % Änderung Pd |
| Vorwerte | 0 | 395 | - | 96 | - |
| 1 | 10 | 241 | -39 | 91 | - 5 |

Wie aus Tabelle II ersichtlich ist, reduzieren die Testsubstanzen die Herzfrequenz, ohne daß im Dosisbereich der herzfrequenzsenkenden Wirkungen der diastolische Blutdruck merklich beeinflußt wird.

Aufgrund der vorstehend beschriebenen pharmakologischen Eigenschaften, insbesondere der ausgeprägten herzfrequenzsenkenden Wirkungen in Kombination mit antiarrhythmischen Eigenschaften, eignen sich die Substanzen zur Prophylaxe und Behandlung von Herzkreislauferkrankungen. Aufgrund ihres günstigen Wirkungsprofils sind die Substanzen auch zur Behandlung von ischämisch beeinflußten Herzkrankheiten geeignet.

Die zu verwendenden Dosen können individuell verschieden sein und variieren naturgemäß je nach Art des zu behandelnden Zustandes, der verwendeten Substanz und der Applikationsform. Zum Beispiel werden parenterale Formulierungen im allgemeinen weniger Wirkstoff enthalten als orale Präparate. Im allgemeinen eignen sich jedoch für Applikationen an größeren Säugetieren, insbesondere Menschen, Arzneiformen mit einem Wirkstoffgehalt von 0,1-10 mg pro Einzeldosis.

Als Heilmittel können die Verbindungen der Formel I und ihre physiologisch verträglichen Säureadditionssalze mit üblichen pharmazeutischen Hilfsstoffen in galenischen Zubereitungen wie z.B. Tabletten, Kapseln, Suppositorien oder Lösungen enthalten sein. Diese galenischen Zubereitungen können nach an sich bekannten Methoden hergestellt werden unter Verwendung üblicher fester Trägerstoffe wie z.B. Milchzucker, Stärke oder Talkum oder flüssiger Verdünnungsmittel wie z.B. Wasser, fetten Ölen oder flüssigen Paraffinen und unter Verwendung von pharmazeutisch üblichen Hilfsstoffen, beispielsweise Tablettensprengmitteln, Lösungsvermittlern oder Konservierungsmitteln.

Die nachfolgenden Beispiele sollen die Erfindung näher erläutern, jedoch deren Umfang in keiner Weise beschränken.

Sofern die in den nachfolgenden Beispielen hergestellten Verbindungen nicht durch ihren Schmelzpunkt charakterisiert sind, werden sie durch die Retentionszeit im Gaschromatographen charakterisiert.

Die Retentionszeitmessung wurde unter folgenden Bedingungen vorgenommen:
Verwendeter Gaschromatograph:
Gaschromatograph der Fa. Hewlett Packard mit der Typenbezeichnung 5750G,
verwendeter Detektor: Flammenionisationsdetektor,
Detektortemperatur: 300 °C,
Injektionstemperatur: 290 °C,
Aufheizgeschwindigkeit von 80 auf 280 °C: 15 °C/min.

Es wurden die folgenden zwei Typen von Säulen verwendet:

| | |
|---|---|
| Säule Typ A *: | 30 m Länge, 0,75 mm Innendurchmesser, Methylsilicon-Innenbeschichtung mit einer Filmdicke von 1 µm; Trägergas Stickstoff, Durchlaufgeschwindigkeit 12 ml/min. |
| Säule Typ B**: | 6 Fuß Länge, 1/8 Zoll Innendurchmesser, gefüllt mit einem Füllmittel auf $SiO_2$-Basis mit einer Korngröße von 80/100***; Trägergas Stickstoff, Durchlaufgeschwindigkeit 22 ml/min. |

Beispiel 1:

7-Cinnamyl-3-n-butyl-9,9-dimethyl-3,7-diazabicyclo[3,3,1]nonan.

1,5 g 3-Butyl-9,9-dimethyl-3,7-diazabicyclo[3,3,1]nonan werden in 20 ml Dichlormethan gelöst und die Lösung wird mit einer Lösung von 1,2 g Cinnamylchlorid in 20 ml Dichlormethan versetzt. Das Reaktionsgemisch wird eine Stunde bei Raumtemperatur reagieren gelassen.

Zur Aufarbeitung und weiteren Reinigung wird der die rohe Titelverbindung enthaltende Rückstand in wäßriger Zitronensäurelösung aufgenommen, wobei die Titelverbindung als zitronensaures Salz in Lösung geht. Zur Abtrennung nicht basischer Verunreinigungen wird die Lösung mit Diäthyläther extrahiert. Anschließend wird die wäßrige Lösung mit verdünnter wäßriger Natronlauge alkalisch gestellt, wobei die Titelverbindung wieder als Base freigesetzt wird. Diese wird mit Diäthyläther extrahiert. Nach Trocknen des Ätherextraktes über Magnesiumsulfat wird die ätherische Lösung filtriert und der Äther abdestilliert. Es werden 1,4 g 7-Cinnamyl-3-n-butyl-9,9-dimethyl-3,7-diazabicyclo[3,3,1]nonan als ölige Base erhalten.
Gaschromatographie: Säule B, Retentionszeit: 6,21 min.

Beispiel 2:

7-Cinnamyl-3-isobutyl-9,9-dimethyl-3,7-diazabicyclo[3,3,1]-nonan.

3g 3-Isobutyl-9,9-dimethyl-3,7-diazabicyclo[3,3,1]nonan werden in 25 ml Dimethylformamid gelöst und die Lösung mit 0,6 g Lithiumamid versetzt und anschließend 60 Minuten lang bei einer Temperatur von 60 °C weitergerührt. Nach dem Abkühlen der Lösung wird tropfenweise eine Lösung von 4,1 g Cinnamylchlorid in 25 ml Dimethylformamid zugegeben und das Reaktionsgemisch weitere 2 Stunden bei 40 °C gerührt.

Zur Aufarbeitung wird das Lösungsmittel abdestilliert und der die Titelverbindung enthaltende Rückstand mit wäßriger Zitronensäurelösung versetzt und wie in Beispiel 1 beschrieben aufgearbeitet. Es werden 3,1 g 7-Cinnamyl-3-isobutyl-9,9-dimethyl-3,7-diazabicyclo[3,3,1]nonan als ölige Base erhalten.
Gaschromatographie: Säule A, Retentionszeit: 12,45 min.

Beispiel 3:

7-Cinnamyl-3-benzyl-9,9-dimethyl-3,7-diazabicyclo[3,3,1]nonan.

A) 3,9 g Zimtsäurechlorid werden in 20 ml Dichlormethan gelöst und zu dieser Lösung wird unter Eiskühlung eine Lösung von 5,5 g 3-Benzyl-9,9-dimethyl-3,7-diazabicyclo[3,3,1]nonan in 10 ml Dichlormethan zugegeben. Anschließend wird das Reaktionsgemisch 3 Stunden bei Raumtemperatur gerührt. Sodann wird zur Aufarbeitung das Lösungsmittel abdestilliert und der das gebildete 7-Cinnamoyl-3-benzyl-9,9-dimethyl-3,7-diazabicyclo[3,3,1]nonan enthaltende Rückstand mit Wasser aufgenommen. Zur Entfernung nichtbasischer Anteile wird mit Essigsäureäthylester extrahiert. Sodann wird die wäßrige Phase mit verdünnter Natronlauge alkalisch gestellt und das Reaktionsprodukt mit Essigester extrahiert. Der Essigesterextrakt wird über Magnesiumsulfat getrocknet, filtriert und das Lösungsmittel abdestilliert.

* = Säule Typ SPB1 der Fa. Supelco

** = Säule Typ 3% OV1 der Fa. Supelco

*** = Supelcoport$^R$ der Fa. Supelco

Als Rückstand erhält man 5 g 7-Cinnamoyl-3-benzyl-9,9-dimethyl-3,7-diazabicyclo[3,3,1]nonan.
Gaschromatographie: Säule A, Retentionszeit: 19,96 min.

B) 0,8 g des vorstehendes Produktes werden in 20 ml Toluol gelöst, und zu der Lösung werden bei Raumtemperatur tropfenweise 0,9 ml einer 3,4 -molaren Lösung von Natriumbis-(2-methoxyäthoxy)-dihydroaluminat (Red-Al$^R$) in Toluol gegeben. Anschließend wird das Reaktionsgemisch weitere 12 Stunden gerührt. Zur Aufarbeitung des die Titelverbindung enthaltenen Reaktionsgemisches werden nacheinander 5 ml Wasser, 5 ml 20 %-ige wäßrige Natronlauge und erneut 15 ml Wasser zugegeben. Von dem gebildeten Aluminatniederschlag wird abfiltriert, und das Filtrat wird mit Essigsäureäthylester extrahiert. Nach Trocknen über Magnesiumsulfat und Einengen der Lösung erhält man als Rückstand 0,5 g 7-Cinnamyl-3-benzyl-9,9-dimethyl-3,7-diazabicyclo[3,3,1]nonan als Öl.
Gaschromatographie: Säule A, Retentionszeit: 15,39 min.

Beispiel 4:

7-(4'-Nitrocinnamyl)-3-cyclopropylmethyl-9,9-tetramethylen-3,7-diazabicyclo[3,3,1]nonan

Zu einer Lösung von 1,24 g 3-Cyclopropylmethyl-9,9-tetramethylen-3,7-diazabicyclo[3,3,1]nonan in 50 ml Methanol werden 0,8 g p-Nitrozimtaldehyd zugegeben und das Reaktionsgemisch eine Stunde bei Raumtemperatur reagieren gelassen. Sodann werden zu dem das gebildete 7-(4'-Nitro-α-hydroxycinnamyl)-3-cyclopropylmethyl-9,9-tetramethylen-3,7-diazabicyclo[3,3,1]nonan enthaltenden Reaktionsgemisch langsam 0,8 g Natriumborhydrid zugegeben und das Reaktionsgemisch weitere 2 Stunden bei Raumtemperatur reagieren gelassen. Anschließend wird das Lösungsmittel abdestilliert und der Rückstand in Methylenchlorid aufgenommen. Die Methylenchloridlösung wird mit Wasser gewaschen, über Magnesiumsulfat getrocknet und eingeengt. Aus dem Rückstand wird das 7-(4'-Nitrocinnamyl)-3-cyclopropylmethyl-9,9-tetramethylen-3,7-diazabicyclo[3,3,1]nonan durch Kugelrohrdestillation unter vermindertem Druck isoliert.
Gaschromatographie: Säule A, Retentionszeit: 19,92 min.

Analog zu den in den vorstehenden Beispielen beschriebenen Verfahren können auch die in der nachstehenden Tabelle 1 angeführten Verbindungen der Formel I erhalten werden.

TABELLE 1

| Beispiel Nr. | $R^1$ | $R^2$ | $R^3$ | $R^4$=cinnamyl subst. durch $R^9$ | $R^{10}$ | $R^{11}$ | Bemerkungen Gaschromat. Retentionszeit RT der Base in min. (Säule A oder B) |
|---|---|---|---|---|---|---|---|
| 5 | Cycloprop-$CH_2$- | $CH_3$- | $CH_3$- | H | H | H | RT: 13,15 (A) |
| 6 | $(CH_3)_2CH$- | $-(CH_2)_4-$ | | H | H | H | RT: 13,87 (A) |
| 7 | $(CH_3)_2CH$-$CH_2$- | $-(CH_2)_4-$ | | H | H | H | RT: 14,81 (A) |
| 8 | Cycloprop-$CH_2$- | $-(CH_2)_4-$ | | H | H | H | RT: 7,14 (B) |
| 9 | Benz- | $-(CH_2)_4-$ | | H | H | H | RT: 18,91 (A) |
| 10 | Cyclohex-$CH_2$- | $CH_3$- | $CH_3$- | H | H | H | RT: 15.04 (A) |
| 11 | n-$C_4H_9$- | $CH_3$- | $CH_3$- | 2-$OCH_3$ | H | H | RT: 13,62 (A) |
| 12 | n-$C_6H_{13}$- | $-(CH_2)_5-$ | | 4-Cl | H | H | RT: 18,82 (A) |
| 13 | n-$C_6H_{13}$- | $-(CH_2)_5-$ | | 3-Cl | 4-Cl | H | RT: 21,69 (A) |
| 14 | n-$C_6H_{13}$- | $-(CH_2)_5-$ | | 4-$NO_2$ | H | H | RT: 23,91 (A) |
| 15 | Cycloprop-$CH_2$- | $-(CH_2)_4-$ | | 3-$CH_3$ | H | H | RT: 15,99 (A) |
| 16 | Cycloprop-$CH_2$- | $-(CH_2)_4-$ | | 4-$CF_3$ | H | H | RT: 15,65 (A) |
| 17 | Cycloprop-$CH_2$- | $-(CH_2)_4-$ | | 3,4,5-tri-$OCH_3$- | | | RT: |

Benz=Benzyl, Cycloprop=Cyclopropyl, Cyclohex=Cyclohexyl

Die verwendeten Ausgangsstoffe wurden nach den folgenden allgemeinen Arbeitsvorschriften hergestellt:

A) Allgemeine Arbeitsvorschrift zur Herstellung von 3,7-disubstituierten 2,4,6,8-Tetraoxo-3,7-diazabicyclo-[3,3,1]nonan-Verbindungen der Formel VII durch Umsetzung von 2,4,6,8-Tetraoxo-3,7-diazabicyclo[3,3,1]-nonan-Verbindungen der Formel V mit Benzylhalogeniden der Formel VI.

a) Umsetzung von N-monosubstituierten Verbindungen der Formel V, worin $R^1$ nicht = H.

Eine Mischung aus 0,1 Mol der Imid-Verbindung der Formel V, 0,2 Mol Kaliumcarbonat und 0,15 Mol Benzylhalogenid der Formel VI in 390 ml Dimethylformamid wird 3 bis 7 Stunden lang am Rückfluß erhitzt. Anschließend wird von dem gebildeten Niederschlag anorganischer Salze abfiltriert und die klare Lösung bis zur Trockne eingeengt. Der verbleibende Rückstand wird in Wasser und Essigester gelöst. Die organische Lösung wird abgetrennt, zweimal mit Wasser gewaschen, über Magnesiumsulfat getrocknet, filtriert und eingeengt. Falls die gebildete Tetraoxo-Verbindung der Formel VII hierbei bereits kristallin anfällt, genügt zur weiteren Reinigung eine einfach Umkristallisierung. Anderenfalls kann es notwendig sein, daß erhaltene Rohprodukt säulenchromatographisch über Kieselgel oder Aluminiumoxid unter Verwendung von Essigester-Hexan-Gemischen als Elutionsmittel zu reinigen.

b) Umsetzung solcher Verbindungen der Formel V, worin $R^1$ = H.

Zur Disubstitution der Verbindungen der Formel V, worin $R^1$ = H durch die Benzylhalogenide der Formel VI wird die vorstehende allgemeine Arbeitsvorschrift zur Monosubstituierung der Verbindungen der Formel V, worin $R^1$ nicht = H, abgewandelt. Anstelle der vorstehend angegebenen Reaktionsmischung wird eine Mischung aus 0,1 Mol der Tetraoxoverbindung der Formel V, 0,25 Mol Kaliumcarbonat und 0,3 Mol Benzylhalogenid der Formel VI in 300 ml Dimethylformamid eingesetzt.

Nach den vorstehenden allgemeinen Arbeitsvorschriften wurden die folgenden in Tabelle A angegebenen Verbindungen hergestellt.

Tabelle A

| Verbindungen der Formel VII | | | | | |
|---|---|---|---|---|---|
| Substanz Nr. | $R^1$ | $R^2$ | $R^3$ | $R^{11}$ | Bemerkungen Fp in °C |
| A1 | n-$C_4H_9$ | $CH_3$ | $CH_3$ | Benz | Fp: 110 |
| A2 | n-$C_4H_9$ | n-$C_3H_7$ | n-$C_3H_7$ | Benz | Öl* |
| A3 | Cyclohex-$CH_2$- | $CH_3$ | $CH_3$ | Benz | Fp: 129-131 |
| A4 | (Cycloprop-$CH_2$- | $CH_3$ | $CH_3$ | Benz | Öl* |
| A5 | $(CH_3)_2CH-CH_2$- | $CH_3$ | $CH_3$ | Benz | Öl* |
| A6 | $(CH_3)_2CH$- | -$(CH_2)_4$- | | Benz | Öl* |
| A7 | Cycloprop-$CH_2$- | -$(CH_2)_4$- | | Benz | Öl* |
| A8 | $(CH_3)_2CH-CH_2$- | -$(CH_2)_4$- | | Benz | Öl* |
| A9 | Cyclohex-$CH_2$- | -$(CH_2)_4$- | | Benz | Öl* |
| A10 | Benz | -$(CH_2)_4$- | | Benz | Öl* |
| A11 | n-$C_6H_{13}$ | -$(CH_2)_5$- | | Benz | Öl* |
| A12 | Benz | $CH_3$ | $CH_3$ | Benz | Fp: 155-157 |
| A13 | Benz | -$(CH_2)_5$- | | Benz | Fp: 150-154 |
| Benz = Benzyl, Cyclohex = Cyclohexyl, Cycloprop = Cyclopropyl | | | | | |

*Öl = wurde als Öl weiterverarbeitet

B) Allgemeine Arbeitsvorschrift zur Reduktion von 2,4,6,8-Tetraoxo-3,7-diazabicyclo 3,3,1 nonan-Verbindungen der Formel VII zu 3,7-Diazabicyclo 3,3,1 nonan-Verbindungen der Formel IV.

In einem Dreihalskolben werden 0,1 Mol Lithiumaluminiumhydrid in 100 ml einer Lösung aus 70 ml absolutem Tetrahydrofuran und 30 ml absolutem Toluol auf eine Ölbadtemperatur von 80 °C erwärmt. Sodann werden langsam 0,025 Mol der Tetraoxoverbindung in 100 ml einer Mischung Tetrahydrofuran/Toluol 70/30 zugetropft. Das Reaktionsgemisch wird 2 bis 4 Stunden lang bei 120 °C reagieren gelassen.

Anschließend wird unter basischen Bedingungen hydrolysiert und mit Methylenchlorid extrahiert. Die organische Phase wird abgetrennt, über Magnesiumsulfat getrocknet und eingeengt. Die gebildeten 3,7-Diazabicyclo[3,3,1]nonan-Verbindungen kristallisieren aus oder werden durch Kugelrohrdestillation unter vermindertem Druck abgetrennt.

Nach dieser allgemeinen Arbeitsvorschrift zur Reduktion mittels Lithiumaluminiumhydrid wurden die in der nachstehenden Tabelle B angegebenen 3,7-Diazabicyclo[3,3,1]nonan-Verbindungen der Formel IV hergestellt.

## T A B E L L E   B

| Substanz IV Nr. | $R^1$ | $R^2$ | $R^3$ | $R^{1'}$ | Bemerkungen: Siedepunkt in °C (0,01 Torr) Fp in °C | Gaschrom. Retentionszeit in min. (Säule A oder B) |
|---|---|---|---|---|---|---|
| B1 | $n-C_4H_9$ | $CH_3$ | $CH_3$ | Benz | Sdp: 170 | |
| B2 | $n-C_4H_9$ | $n-C_3H_7$ | $n-C_3H_7$ | Benz | Sdp: 200 | |
| B3 | Cyclohex-$CH_2$- | $CH_3$ | $CH_3$ | Benz | Sdp: 170 | 13,23 (A) |
| B4 | Cycloprop-$CH_2$- | $CH_3$ | $CH_3$ | Benz | | 11,54 (A) |
| B5 | $(CH_3)_2CH-CH_2$- | $CH_3$ | $CH_3$ | Benz | | 9,85 (B) |
| B6 | $(CH_3)_2CH$- | $-(CH_2)_4-$ | | Benz | | 10,84 (A) |
| B7 | Cycloprop-$CH_2$- | $-(CH_2)_4-$ | | Benz | | 6,29 (B) |
| B8 | $(CH_3)_2CH-CH_2$ | $-(CH_2)_4-$ | | Benz | | 12,67 (A) |
| B9 | Cyclohex-$CH_2$- | $-(CH_2)_4-$ | | Benz | Fp.: 58 | 7,23 (B) |
| B10 | Benz- | $-(CH_2)_4-$ | | Benz | Fp.: 54 | 7,24 (B) |
| B11 | $n-C_6H_{13}$ | $-(CH_2)_5-$ | | Benz | | 14,70 (A) |
| B12 | Benz- | $CH_3$ | $CH_3$ | Benz | Fp.: 58 | 13,47 (A) |
| B13 | Benz- | $-(CH_2)_5-$ | | Benz | Fp.: 60 | 16,66 (A) |

Cycloprop = Cyclopropyl, Benz = Benzyl, Cyclohex = Cyclohexyl,

C) Allgemeine Arbeitsvorschrift zur Debenzylierung der 3,7-disubstituierten 3,7-Diazabicyclo[3,3,1]nonan-Verbindungen der Formel IV zu N-monosubstituierten 3,7-Diazabicyclo[3,3,1]nonan-Verbindungen der Formel II.

0,2 Mol einer Verbindung der Formel IV werden in 600 ml Äthanol unter Zusatz von 5 ml Eisessig gelöst und die Lösung wird mit 10 g Palladium/ Kohle-Katalysator versetzt. Das Reaktionsgemisch wird

bei Raumtemperatur unter einem Wasserstoffdruck von 5 Atmosphären ca. 6 Stunden hydriert. Nach Beendigung der Hydrierung wird die Lösung von dem Katalysator abgetrennt und eingeengt. Die gebildeten Verbindungen der Formel II können mit Hilfe von Kugelrohrdestillation unter vermindertem Druck weiter gereinigt werden.

Nach der vorstehenden allgemeinen Vorschrift zur Debenzylierung wurden die in der folgenden Tabelle C angegebenen 3,7-Diazabicyclo[3,3,1]nonan-Verbindungen der Formel II hergestellt.

## TABELLE C

Verbindungen der Formel II

| Sub-stanz IV Nr. | $R^1$ | $R^2$ | $R^3$ | Siedepunkt in °C (0,01 Torr) Fp in °C | Bemerkungen Gaschrom. Retentionszeit in min. (Säule A oder B) |
|---|---|---|---|---|---|
| C1 | $n-C_4H_9-$ | $CH_3$ | $CH_3$ | Sdp: 145 | |
| C2 | $n-C_4H_9-$ | $n-C_3H_7$ | $n-C_3H_7$ | Sdp: 220 (1,5 Torr) | |
| C3 | Cyclohex-$CH_2$- | $CH_3$ | $CH_3$ | n. b. | |
| C4 | Cycloprop-$CH_2$- | $CH_3$ | $CH_3$ | | 7,74 (A) |
| C5 | $(CH_3)_2CH-CH_2-$ | $CH_3$ | $CH_3$ | | 5,78 (B) |
| C6 | Cycloprop-$CH_2$- | $-(CH_2)_4-$ | | 2WS: Fp. 75 | 4,59 (B) |
| C7 | $(CH_3)_2CH-$ | $-(CH_2)_4-$ | | 2WS: Fp. 115 | 8,71 (A) |
| C8 | $(CH_3)_2CH-CH_2$ | $-(CH_2)_4-$ | | | 9,32 (A) |
| C9 | Cyclohex-$CH_2$- | $-(CH_2)_4-$ | | 1HCl: Fp. 185 | 11,83 (A) |
| C10 | Benz | $-(CH_2)_4-$ | | | 12,38 (A) |
| C11 | $n-C_6H_{13}-$ | $-(CH_2)_5-$ | | | 11,58 (A) |
| C12 | Benz | $CH_3$ | $CH_3$ | Sdp: 180 | |
| C13 | Benz | $-(CH_2)_5-$ | | | 12,62 (A) |

Cycloprop = Cyclopropyl, WS = Hydrogentartrat, Benz = Benzyl,
Cyclohex = Cyclohexyl, HCl = Hydrochlorid
n.b. = nicht bestimmt, ohne Reinigung weiterverarbeitet

Beispiel I:

7-Cinnamyl-3-isobutyl-9,9-dimethyl-3,7-diazabicyclo[3,3,1]nonan enthaltende Tabletten.

Es werden Tabletten in folgender Zusammensetzung pro Tablette hergestellt:

| | |
|---|---|
| 7-Cinnamyl-3-isobutyl-9,9-dimethyl-3,7-diazabicyclo[3,3,1]nonan | 20 mg |
| Maisstärke | 60 mg |
| Milchzucker | 135 mg |
| Gelatine (als 10%-ige Lösung) | 6 mg |

Der Wirkstoff, die Maisstärke und der Milchzucker werden mit der 10 %-igen Gelatine-Lösung eingedickt. Die Paste wird zerkleinert und das entstandene Granulat wird auf ein geeignetes Blech gebracht und getrocknet. Das getrocknete Granulat wird durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann zu Tabletten von 240 mg verpreßt.

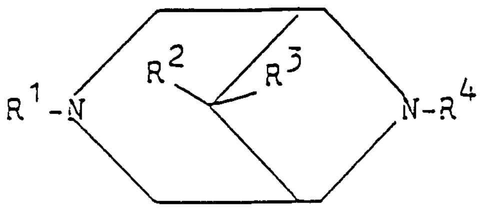

I

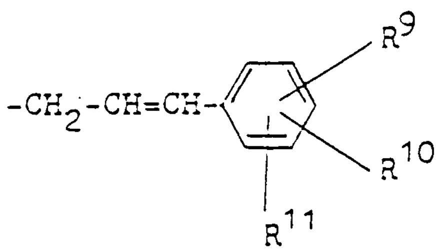

a

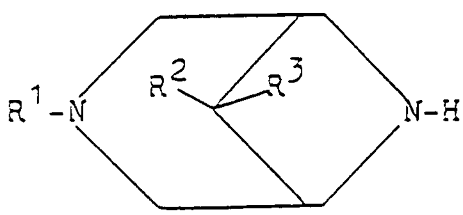

II

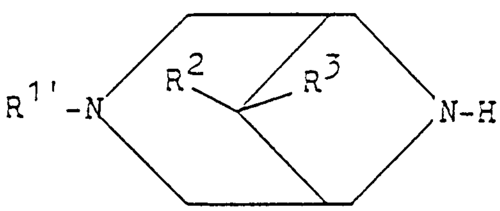

IIa

$R^4$-X III

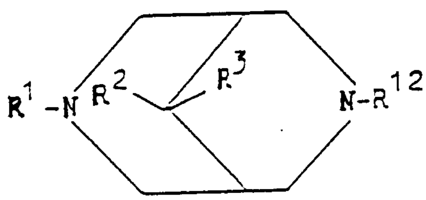

IV

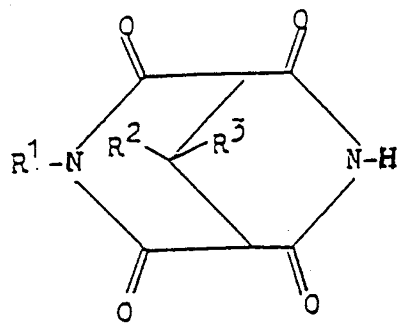

V

$R^{12}$-Hal VI

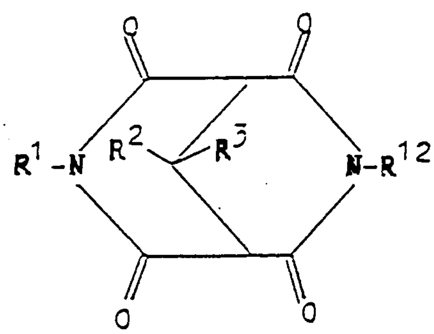

VII

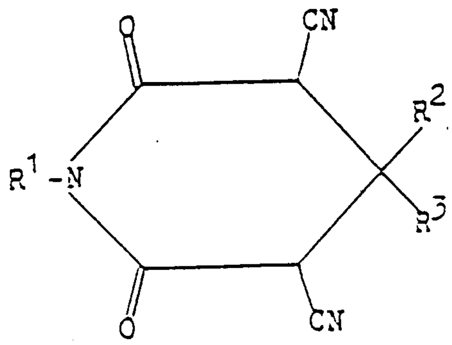

VIII

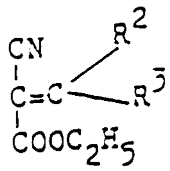

IX

$R^1$-NH-CO-$CH_2$-CN X

$R^{1'}$-X XI

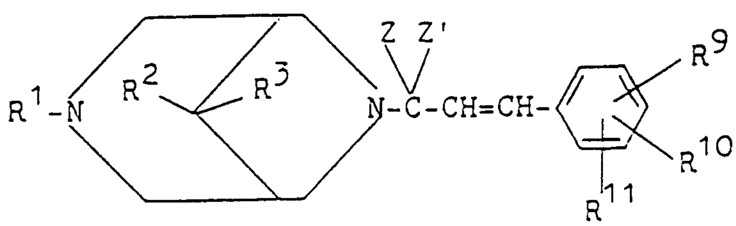

XIV

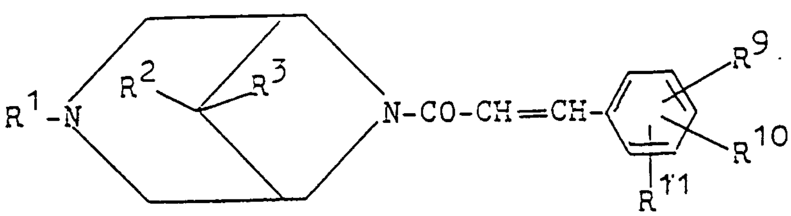

XIVa

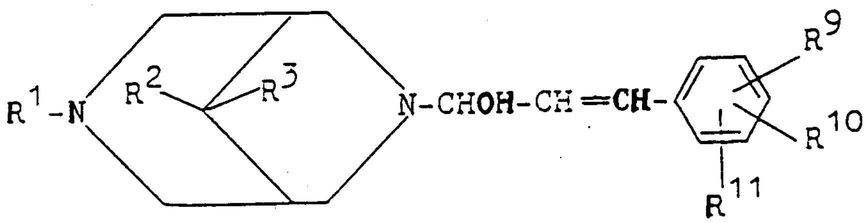

XIVb

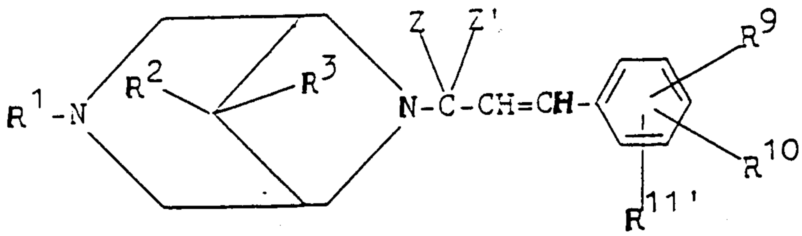

XIV'

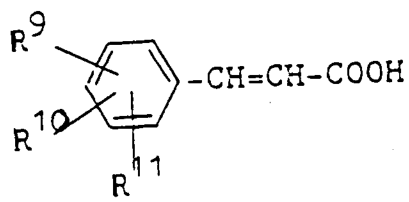

XV

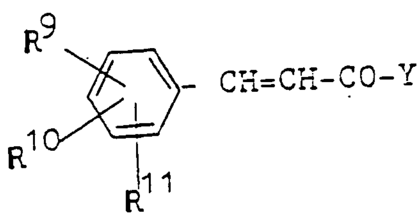

XVa

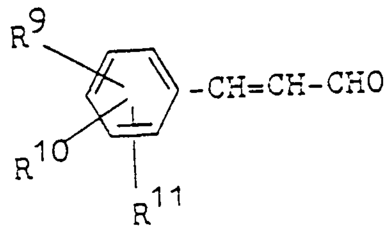

XVI

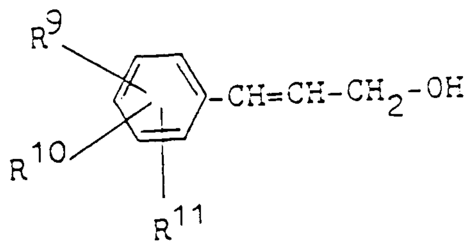

XVII

## Patentansprüche
## Patentansprüche für folgende Vertragsstaaten : BE, CH, DE, FR, GB, IT, LI, NL

**1.** 3,7-Diazabicyclo[3,3,1]nonan-Verbindungen der allgemeinen Formel I

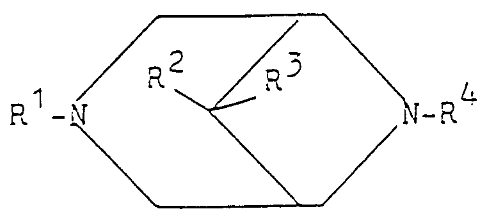

I

worin

| | |
|---|---|
| $R^1$ | eine Alkylgruppe mit 1-6 Kohlenstoffatomen, eine Cycloalkylalkylgruppe mit 4-9 Kohlenstoffatomen oder Benzyl bedeutet, |
| $R^2$ | Alkyl mit 1-4 Kohlenstoffatomen bedeutet und |
| $R^3$ | Alkyl mit 1-4 Kohlenstoffatomen bedeutet oder |
| $R^2$ und $R^3$ | gemeinsam eine Alkylenkette mit 3-6 Kohlenstoffatomen bilden, und |
| $R^4$ | eine Cinnamylgruppe der allgemeinen Formel a |

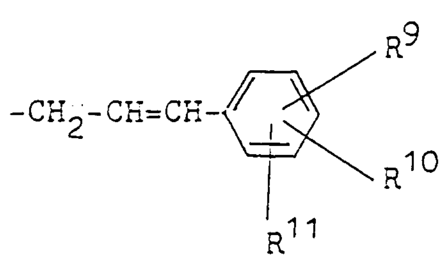

a

bedeutet, worin

$R^9$ Wasserstoff, Halogen, Alkyl mit 1-4 Kohlenstoffatomen, Alkoxy mit 1-4 Kohlenstoffatomen oder Hydroxy bedeutet,

$R^{10}$ Wasserstoff, Halogen, Alkoxy mit 1-4 Kohlenstoffatomen, Alkyl mit 1-4 Kohlenstoffatomen, Hydroxy oder, falls $R^9$ Wasserstoff ist, auch Trifluormethyl oder Nitro bedeutet, und

$R^{11}$ Wasserstoff oder, falls $R^9$ und $R_{10}$ Alkoxy mit 1-4 Kohlenstoffatomen sind, auch Alkoxy mit 1-4 Kohlenstoffatomen bedeutet,

und deren Säureadditionssalze.

**2.** Verbindungen gemäß Anspruch 1, worin $R^1$ eine Alkylgruppe mit 1-6 Kohlenstoffatomen oder eine Cycloalkylalkylgruppe mit 4-9 Kohlenstoffatomen bedeutet.

**3.** Verbindungen gemäß Anspruch 2, worin die Cycloalkylalkylgruppe 4-7 Kohlenstoffatome enthält.

**4.** Verbindungen gemäß Anspruch 1, worin $R^{11}$ Wasserstoff bedeutet.

**5.** Arzneimittel, enthaltend eine pharmakologisch wirksame Menge einer 3,7-Diazabicyclo[3,3,1]nonan-Verbindung gemäß Anspruch 1, und übliche pharmazeutische Hilfs- und/oder Trägerstoffe.

**6.** Verfahren zur Herstellung von 3,7-Diazabicyclo[3,3,1]nonan-Verbindungen der allgemeinen Formel I

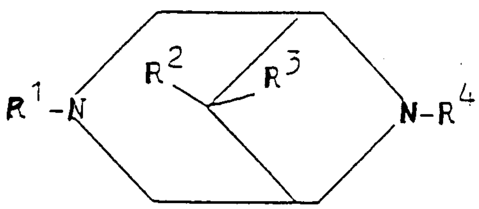

I

worin $R^1$, $R^2$, $R^3$ und $R^4$ die in Anspruch 1 angegebene Bedeutung besitzen,
und deren Säureadditionssalzen,
dadurch gekennzeichnet, daß man

a) Verbindungen der allgemeinen Formel II

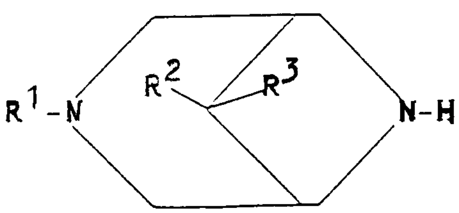

II

worin $R^1$, $R^2$ und $R^3$ obige Bedeutung besitzen, mit Verbindungen der allgemeinen Formel III

$R^4$-X III

worin $R^4$ die für $R^4$ oben angegebene Bedeutung besitzt, wobei jedoch allfällige freie Hydroxygruppen mit einer Schutzgruppe versehen sind, und X eine aminolytisch abspaltbare Gruppe bedeutet, umsetzt oder

b) Verbindungen der allgemeinen Formel XIV

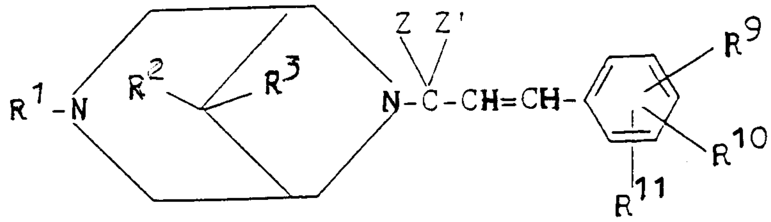

XIV

worin $R^1$, $R^2$, $R^3$ und $R^{11}$ obige Bedeutung besitzen und $R^9$ und $R^{10}$ die oben für $R^9$ und $R^{10}$ angebenene Bedeutung besitzen, wobei jedoch allfällige freie Hydroxygruppen mit einer Schutzgruppe versehen sind, und Z und Z' gemeinsam Sauerstoff bedeuten oder Z für Hydroxy und Z' für Wasserstoff stehen, reduziert,
und anschließend allfällige Hydroxyschutzgruppen wieder abspaltet, und gewünschtenfalls freie Verbindungen der Formel I in ihre Säureadditionssalze überführt oder die Säureadditionssalze in die freien Verbindungen der Formel I überführt.

**7.** Verbindungen der allgemeinen Formel XIV'

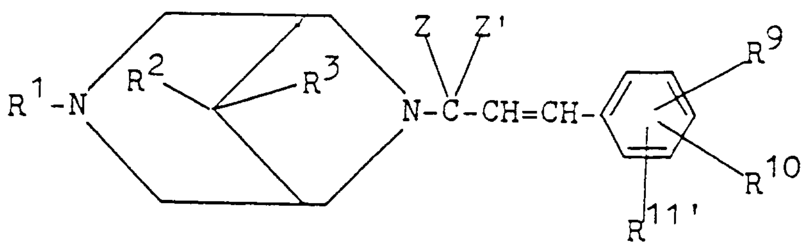

XIV'

worin $R^1$, $R^2$, $R^3$, $R^9$ und $R^{10}$ die in Anspruch 1 angegebene Bedeutung besitzen,

$R^{11'}$ Wasserstoff oder, falls $R^9$ und $R^{10}$ Alkoxy mit 1-4 Kohlenstoffatomen sind und $R^2$ und $R^3$ Alkyl mit 1-4 Kohlenstoffatomen sind, auch Alkoxy mit 1-4 Kohlenstoffatomen bedeutet, und

Z und Z' gemeinsam Sauerstoff bedeuten oder Z für Hydroxy und Z' für Wasserstoff stehen,

und deren Säureadditionssalze.

**Patentansprüche für folgenden Vertragsstaat : ES**

**1.** Verfahren zur Herstellung von 3,7-Diazaoicyclo[3,3,1]nonan-Verbindungen der allgemeinen Formel I

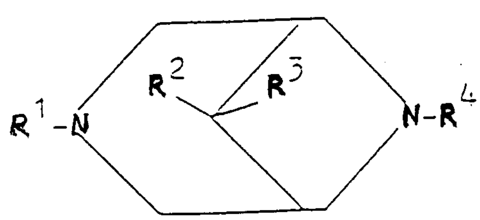

I

worin

$R^1$ eine Alkylgruppe mit 1-6 Kohlenstoffatomen, eine Cycloalkylalkylgruppe mit 4-9 Kohlenstoffatomen oder Benzyl bedeutet,

$R^2$ Alkyl mit 1-4 Kohlenstoffatomen bedeutet und

$R^3$ Alkyl mit 1-4 Kohlenstoffatomen bedeutet oder

$R^2$ und $R^3$ gemeinsam eine Alkylenkette mit 3-6 Kohlenstoffatomen bilden, und

$R^4$ eine Cinnamylgruppe der allgemeinen Formel a

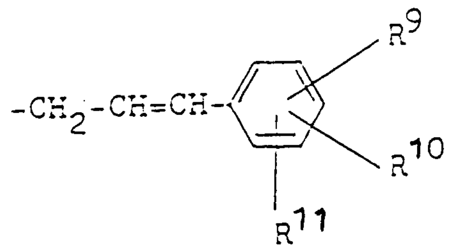

a

bedeutet, worin

$R^9$ Wasserstoff, Alkyl mit 1-4 Kohlenstoffatomen, Halogen, Alkoxy mit 1-4 Kohlenstoffatomen oder Hydroxy bedeutet, und

$R^{10}$ Wasserstoff, Halogen, Alkoxy mit 1-4 Kohlenstoffatomen, Alkyl mit 1-4 Kohlenstoffatomen, Hydroxy oder, falls $R^9$ Wasserstoff ist, auch Trifluormethyl oder Nitro bedeutet, und

$R^{11}$ Wasserstoff oder, falls $R^9$ und $R^{10}$ Alkoxy mit 1-4 Kohlenstoffatomen sind, auch Alkoxy mit 1-4 Kohlenstoffatomen bedeutet,

und deren Säureadditionssalzen,

dadurch gekennzeichnet, daß man

a) Verbindungen der allgemeinen Formel II

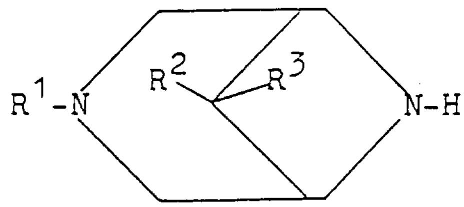

II

worin $R^1$, $R^2$ und $R^3$ obige Bedeutung besitzen, mit Verbindungen der allgemeinen Formel III

$R^4$-X III

worin $R^4$ die für $R^4$ oben angegebene Bedeutung besitzt, wobei jedoch allfällige freie Hydroxygruppen mit einer Schutzgruppe versehen sind, und X eine aminolytisch abspaltbare Gruppe bedeutet, umsetzt oder

b) Verbindungen der allgemeinen Formel XIV

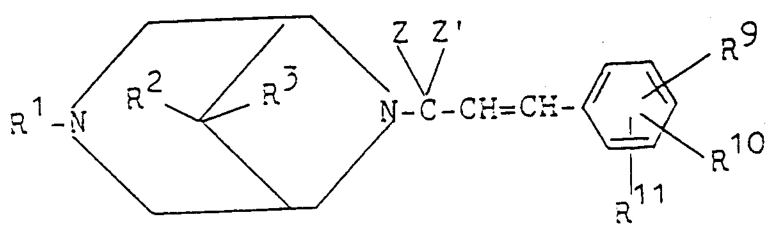

XIV

worin $R^1$, $R^2$, $R^3$ und $R^{11}$ obige Bedeutung besitzen und $R^9$ und $R^{10}$ die oben für $R^9$ und $R^{10}$ angebenene Bedeutung besitzen, wobei jedoch allfällige freie Hydroxygruppen mit einer Schutzgruppe versehen sind, und Z und Z' gemeinsam Sauerstoff bedeuten oder Z für Hydroxy und Z' für Wasserstoff stehen, reduziert,

und anschließend allfällige Hydroxyschutzgruppen wieder abspaltet, und gewünschtenfalls freie Verbindungen der Formel I in ihre Säureadditionssalze überführt oder die Säureadditionssalze in die freien Verbindungen der Formel I überführt.

**2.** Verfahren nach Anspruch 1 dadurch gekennzeichnet, daß Verbindungen, worin $R^1$ eine Alkylgruppe mit 1-6 Kohlenstoffatomen oder eine Cycloalkylalkylgruppe mit 4-9 Kohlenstoffatomen bedeutet, hergestellt

werden.

**3.** Verfahren nach Anspurch 2 dadurch gekennzeichnet, daß die Cycloalkylgruppe 4-7 Kohlenstoffatome enthält.

**4.** Verfahren nach Anspruch 1 dadurch gekennzeichnet, daß Verbindungen, worin $R^{11}$ Wasserstoff bedeutet, hergestellt werden.

**5.** Verfahren zur Herstellung von Verbindungen der allgemeinen Formel XIV'

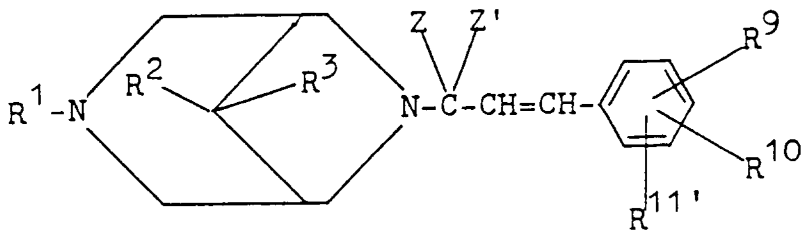

XIV'

worin

| | |
|---|---|
| $R^1$, $R^2$, $R^3$, $R^9$ und $R^{10}$ | die in Anspruch 1 angegebene Bedeutung besitzen, |
| $R^{11'}$ | Wasserstoff oder, falls $R^9$ und $R^{10}$ Alkoxy mit 1-4 Kohlenstoffatomen sind und $R^2$ und $R^3$ Alkyl mit 1-4 Kohlenstoffatomen sind, auch Alkoxy mit 1-4 Kohlenstoffatomen bedeutet, und |
| Z und Z' | gemeinsam Sauerstoff bedeuten oder Z für Hydroxy und Z' für Wasserstoff stehen, |

und deren Säureadditionssalzen, dadurch gekennzeichnet, daß man

a) zur Herstellung von Verbindungen der allgemeinen Formel XIV'a

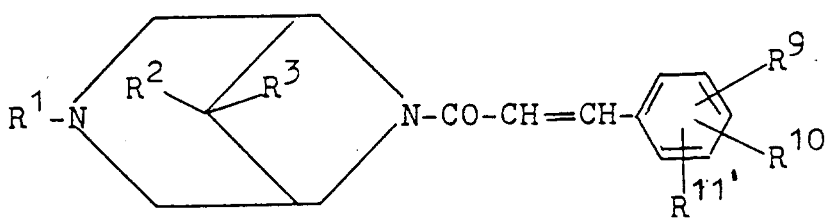

XIV'a

worin $R^1$, $R^2$, $R^3$, $R^9$, $R^{10}$ und $R^{11'}$ obige Bedeutung besitzen, Säuren der allgemeinen Formel XV'

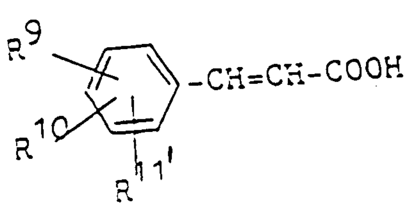

XV'

worin $R^9$, $R^{10}$ und $R^{11'}$ obige Bedeutung besitzen, oder deren reaktionsfähige Säurederivate mit Verbindungen der allgemeinen Formel II

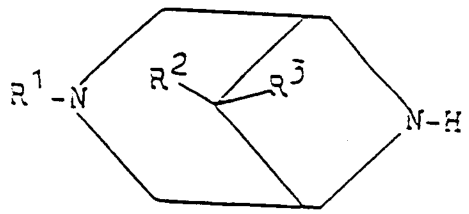

II

worin $R^1$, $R^2$ und $R^3$ obige Bedeutung besitzen, umsetzt, und

b) zur Herstellung von Verbindungen der allgemeinen Formel XIV'b

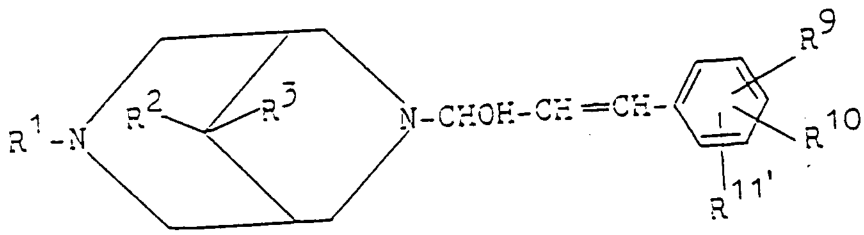

XIV'b

worin $R^1$, $R^2$, $R^3$, $R^9$, $R^{10}$ und $R^{11'}$ obige Bedeutung besitzen, Verbindungen der Formel II mit einem Aldehyd der Formel XVI'

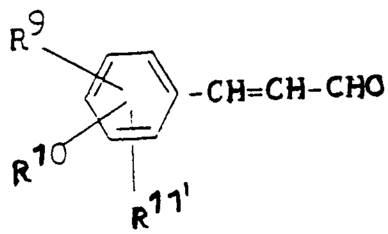

XVI'

worin $R^9$, $R^{10}$ und $R^{11}$ obige Bedeutung besitzen, umsetzt.

## Claims

**Claims for the following Contracting States : BE, CH, DE, FR, GB, IT, LI, NL**

**1.** 3,7-diazabicyclo[3,3,1]nonane compounds of the general Formula I,

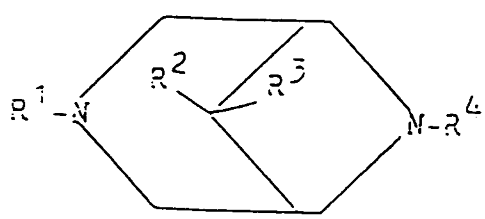

I

wherein

| | |
|---|---|
| $R^1$ | is an alkyl group with 1-6 carbon atoms, a cycloalkylalkyl group with 4-9 carbon atoms or benzyl, |
| $R^2$ | is alkyl with 1-4 carbon atoms and |
| $R^3$ | is alkyl with 1-4 carbon atoms or |
| $R^2$ and $R^3$ | together form an alkylene chain with 3-6 carbon atoms, and |
| $R^4$ | is a cinnamyl group of the general Formula a, |

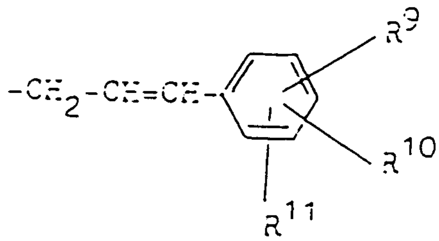

a

wherein

$R^9$ is hydrogen, halogen, alkyl with 1-4 carbon atoms, alkoxy with 1-4 carbon atoms or hydroxy,

$R^{10}$ is hydrogen, halogen, alkoxy with 1-4 carbon atoms, alkyl with 1-4 carbon atoms, hydroxy or, if $R^9$ is hydrogen, also trifluoromethyl or nitro, and

$R^{11}$ is hydrogen, or, if $R^9$ and $R^{10}$ are alkoxy with 1-4 carbon atoms, also alkoxy with 1-4 carbon atoms,

and their acid addition salts.

**2.** Compounds according to Claim 1, wherein $R^1$ is an alkyl group with 1-6 carbon atoms or a cycloalkylalkyl group with 4-9 carbon atoms.

**3.** Compounds according to Claim 2, wherein the cycloalkylalkyl group contains 4-7 carbon atoms.

**4.** Compounds according to Claim 1, wherein $R^{11}$ is hydrogen.

**5.** A medicament, containing a pharmacologically effective quantity of a 3,7-diazabicyclo[3,3,1]nonane compound according to Claim 1, and conventional pharmaceutical auxiliaries and/or carriers.

**6.** A method for the production of 3,7-diazabicyclo[3,3,1]nonane compounds of the general Formula I,

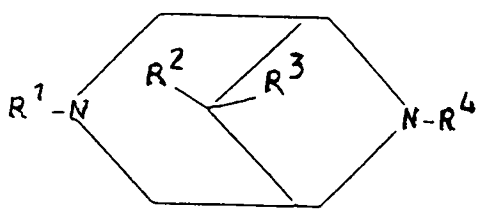

I

wherein

$R^1$, $R^2$, $R^3$ and $R^4$ have the meanings given in Claim 1,

and their acid addition salts,

characterised in that

a) compounds of the general Formula II,

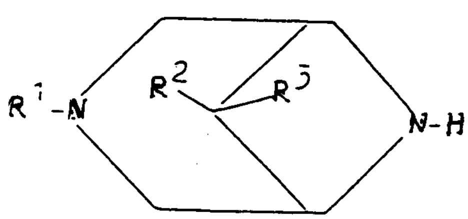

II

wherein $R^1$, $R^2$ and $R^3$ have the above meanings, are reacted with compounds of the general Formula III,

$R^4$-X III

wherein $R^4$ has the meaning given above for $R^4$, but with any free hydroxy groups being provided with a protective group, and X is an aminolytically cleavable group, or
b) compounds of the general Formula XIV,

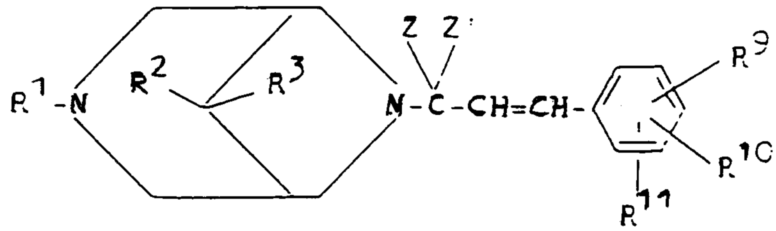

XIV

wherein $R^1$, $R^2$, $R^3$ and $R^{11}$ have the above meanings and $R^9$ and $R^{10}$ have the meanings given above for $R^9$ and $R^{10}$, but with any free hydroxy groups being provided with a protective group, and Z and Z' together are oxygen, or Z stands for hydroxy and Z' for hydrogen, are reduced,
and subsequently any hydroxy protective groups are split off again, and if desired free compounds of Formula I are converted into their acid addition salts or the acid addition salts are converted into the free compounds of Formula I.

**7.** Compounds of the general Formula XIV',

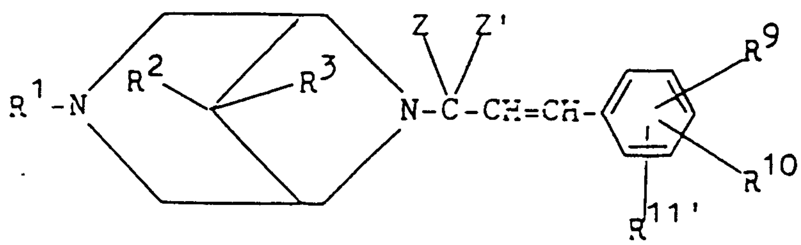

XIV'

wherein $R^1$, $R^2$, $R^3$, $R^9$ and $R^{10}$ have the meanings given in Claim 1,

| | |
|---|---|
| $R^{11'}$ | is hydrogen or, if $R^9$ and $R^{10}$ are alkoxy with 1-4 carbon atoms and $R^2$ and $R^3$ are alkyl with 1-4 carbon atoms, is also alkoxy with 1-4 carbon atoms, and |
| Z and Z' | together are oxygen or Z stands for hydroxy and Z' for hydrogen, |

and their acid addition salts.

**Claims for the following Contracting State : ES**

**1.** A method for the production of 3,7-diazabicyclo[3,3,1]nonane compounds of the general Formula I,

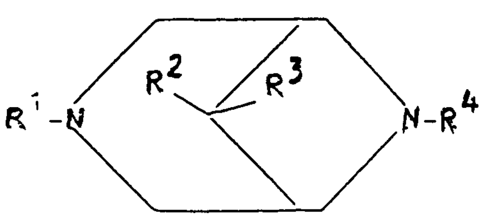

I

wherein

| | |
|---|---|
| $R^1$ | is an alkyl group with 1-6 carbon atoms, a cycloalkylalkyl group with 4-9 carbon atoms or benzyl, |
| $R^2$ | is alkyl with 1-4 carbon atoms and |
| $R^3$ | is alkyl with 1-4 carbon atoms or |

$R^2$ and $R^3$ together form an alkylene chain with 3-6 carbon atoms, and

$R^4$ is a cinnamyl group of the general Formula a,

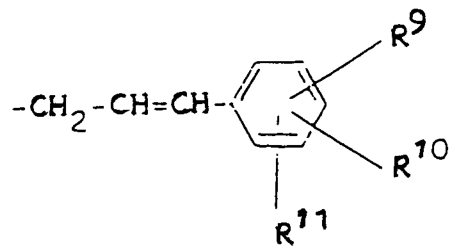

a

wherein

$R^9$ is hydrogen, alkyl with 1-4 carbon atoms, halogen, alkoxy with 1-4 carbon atoms or hydroxy, and

$R^{10}$ is hydrogen, halogen, alkoxy with 1-4 carbon atoms, alkyl with 1-4 carbon atoms, hydroxy or, if $R^9$ is hydrogen, also trifluoromethyl or nitro, and

$R^{11}$ is hydrogen, or, if $R^9$ and $R^{10}$ are alkoxy with 1-4 carbon atoms, also alkoxy with 1-4 carbon atoms,

and their acid addition salts,
characterised in that

a) compounds of the general Formula II,

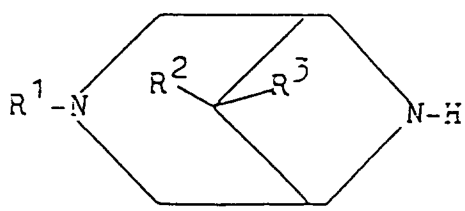

II

wherein $R^1$, $R^2$ and $R^3$ have the above meanings, are reacted with compounds of the general Formula III,

$R^4$-X III

wherein $R^4$ has the meaning given above for $R^4$, but with any free hydroxy groups being provided with a protective group, and X is an aminolytically cleavable group, or

b) compounds of the general Formula XIV,

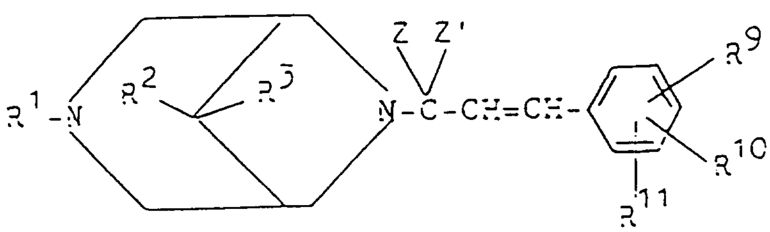

XIV

wherein $R^1$, $R^2$, $R^3$ and $R^{11}$ have the above meanings and $R^9$ and $R^{10}$ have the meanings given above for $R^9$ and $R^{10}$, but with any free hydroxy groups being provided with a protective group, and Z and Z' together are oxygen, or Z stands for hydroxy and Z' for hydrogen, are reduced,
and subsequently any hydroxy protective groups are split off again, and if desired free compounds of Formula I are converted into their acid addition salts or the acid addition salts are converted into the free compounds of Formula I.

2. A method according to Claim 1, characterised in that compounds wherein $R^1$ is an alkyl group with 1-6 carbon atoms or a cycloalkylalkyl group with 4-9 carbon atoms are produced.

3. A method according to Claim 2, characterised in that the cycloalkylalkyl group contains 4-7 carbon atoms.

4. A method according to Claim 1, characterised in that compounds wherein $R^{11}$ is hydrogen are produced.

5. A method for producing compounds of the general Formula XIV',

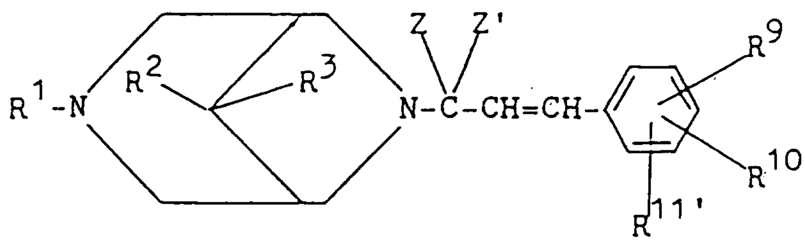

XIV'

wherein

| | |
|---|---|
| $R^1$, $R^2$, $R^3$, $R^9$ and $R^{10}$ | have the meanings given in Claim 1, |
| $R^{11'}$ | is hydrogen or, if $R^9$ and $R^{10}$ are alkoxy with 1-4 carbon atoms and $R^2$ and $R^3$ are alkyl with 1-4 carbon atoms, is also alkoxy with 1-4 carbon atoms, and |
| Z and Z' | together are oxygen or Z stands for hydroxy and Z' for hydrogen, |

and their acid addition salts, characterised in that

a) to produce compounds of the general formula XIV'a,

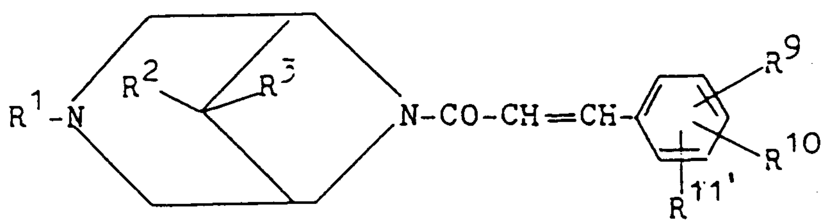

XIV'a

wherein $R^1$, $R^2$, $R^3$, $R^9$, $R^{10}$ and $R^{11'}$ have the above meanings, acids of the general formula XVI',

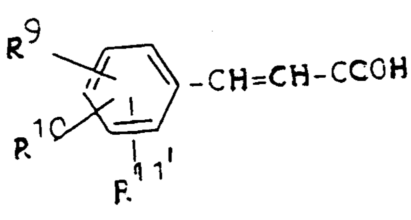

XV'

wherein $R^9$, $R^{10}$ and $R^{11'}$ have the above meanings, or their reactive acid derivatives are reacted with compounds of the general formula II,

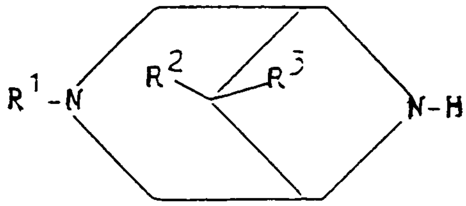

II

wherein $R^1$, $R^2$ and $R^3$ have the above meanings, and
b) to produce compounds of the general formula XIV'b,

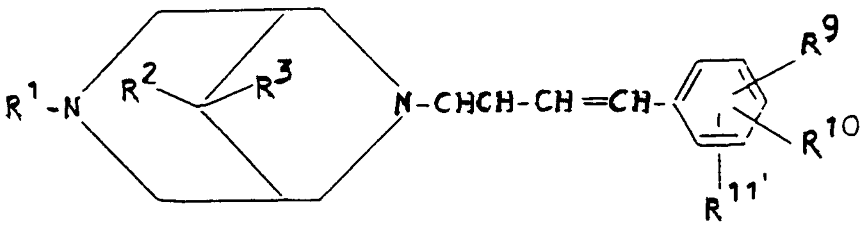

XIV'b

wherein $R^1$, $R^2$, $R^3$, $R^9$, $R^{10}$ and $R^{11'}$ have the above meanings, compounds of Formula II are reacted with an aldehyde of Formula XVI',

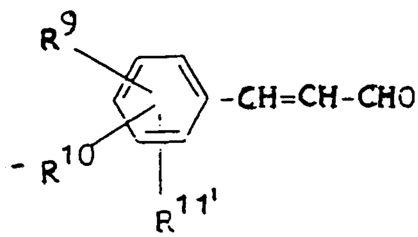

XVI'

wherein $R^9$, $R^{10}$ and $R^{11}$ have the above meanings.

## Revendications

**Revendications pour les Etats contractants suivants : BE, CH, DE, FR, GB, IT, LI, NL**

**1.** Composés de type 3,7-diazabicyclo[3.3.1]nonane, de formule générale I

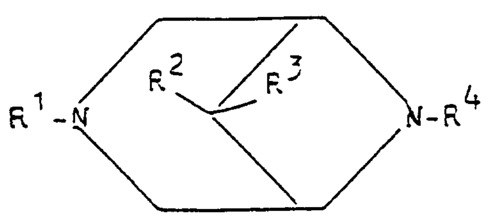

I

dans laquelle

| | |
|---|---|
| $R^1$ | représente un groupe alkyle ayant de 1 à 6 atomes de carbone, un groupe cycloalkylalkyle ayant de 4 à 9 atomes de carbone ou le groupe benzyle, |
| $R^2$ | représente un groupe alkyle ayant de 1 à 4 atomes de carbone et |
| $R^3$ | représente un groupe alkyle ayant de 1 à 4 atomes de carbone, ou |
| $R^2$ et $R^3$ | forment ensemble une chaîne alkylène ayant de 3 à 6 atomes de carbone, et |
| $R^4$ | représente un groupe cinnamyle de formule générale a |

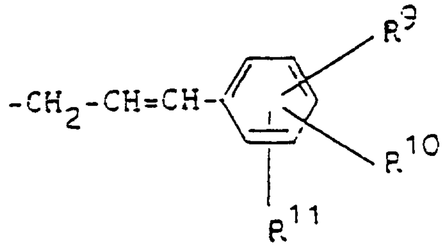

a

dans laquelle

$R^9$ représente un atome d'hydrogène ou d'halogène ou un groupe alkyle ayant de 1 à 4 atomes de carbone, alcoxy ayant de 1 à 4 atomes de carbone ou hydroxy,

$R^{10}$ représente un atome d'hydrogène ou d'halogène ou un groupe alcoxy ayant de 1 à 4 atomes de carbone, alkyle ayant de 1 à 4 atomes de carbone, hydroxy ou, lorsque $R^9$ est un atome d'hydrogène, également le groupe trifluorométhyle ou nitro, et

$R^{11}$ représente un atome d'hydrogène ou, lorsque $R^9$ et $R^{10}$ sont des groupes alcoxy ayant de 1 à 4 atomes de carbone, représente également un groupe alcoxy ayant de 1 à 4 atomes de carbone,

et leurs sels d'addition avec des acides.

2. Composés selon la revendication 1, dans lesquels $R^1$ représente un groupe alkyle ayant de 1 à 6 atomes de carbone ou un groupe cycloalkylalkyle ayant de 4 à 9 atomes de carbone.

3. Composés selon la revendication 2, dans lesquels le groupe cycloalkylalkyle contient de 4 à 7 atomes de carbone.

4. Composés selon la revendication 1, dans lesquels $R^{11}$ représente un atome d'hydrogène.

5. Médicament contenant une quantité pharmacologiquement active d'un composé de type 3,7-diazabicyclo[3.3.1]nonane selon la revendication 1, et des adjuvants et/ou véhicules pharmaceutiques usuels.

6. Procédé de préparation de composés de type 3,7-diazabicyclo[3.3.1]nonane de formule générale I

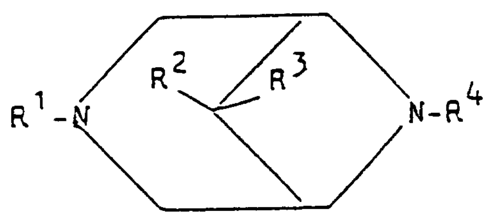

I

dans laquelle $R^1$, $R^2$, $R^3$ et $R^4$ ont les significations données dans la revendication 1,
et de leurs sels d'addition avec des acides,
caractérisé en ce que
a) on fait réagir des composés de formule générale II

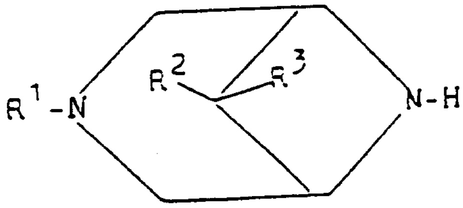

II

dans laquelle $R^1$, $R^2$ et $R^3$ ont les significations données plus haut, avec des composés de formule générale III

$R^4$-X III

dans laquelle $R^4$ a la signification donnée plus haut pour $R^4$, des groupes hydroxy éventuellement libres étant cependant munis d'un groupe protecteur, et X représente un groupe séparable par aminolyse, ou

b) on réduit des composés de formule générale XIV

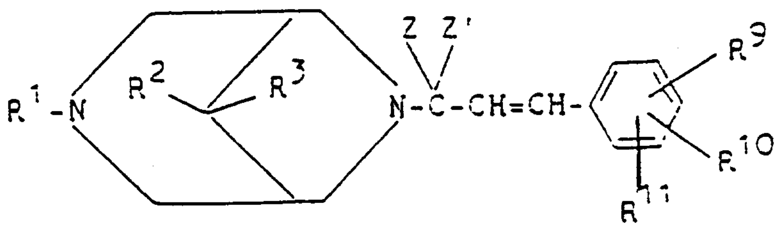

XIV

dans laquelle $R^1$, $R^2$, $R^3$ et $R^{11}$ ont les significations données plus haut, et $R^9$ et $R^{10}$ ont les significations données plus haut pour $R^9$ et $R^{10}$, des groupes hydroxy éventuellement libres étant toutefois munis d'un groupe protecteur, et Z et Z' représentant ensemble un atome d'oxygène, ou Z représente le groupe hydroxy et Z' représente un atome d'hydrogène, et on élimine ensuite les éventuels groupes protecteurs de fonction hydroxy et, si on le désire, on convertit des composés libres de formule I en leurs sels d'addition avec des acides ou les sels d'addition avec des acides en composés libres de formule I.

**7.** Composés de formule générale XIV'

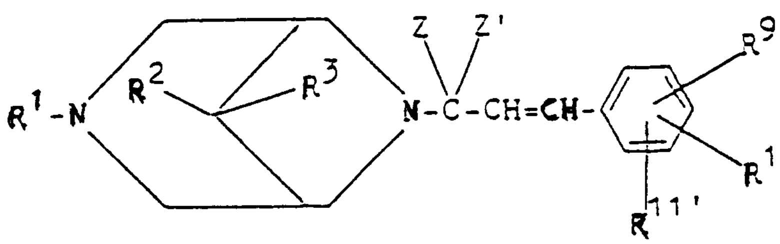

XIV'

dans laquelle $R^1$, $R^2$, $R^3$, $R^9$ et $R^{10}$ ont les significations données dans la revendication 1,

$R^{11'}$ représente un atome d'hydrogène, ou, lorsque $R^9$ et $R^{10}$ sont des groupes alcoxy ayant de 1 à 4 atomes de carbone et $R^2$ et $R^3$ sont des groupes alkyle ayant de 1 à 4 atomes de carbone, représente également un groupe alcoxy ayant de 1 à 4 atomes de carbone, et

Z et Z' représentent ensemble un atome d'oxygène, ou Z représente le groupe hydroxy et Z' représente un atome d'hydrogène,

et leurs sels d'addition avec des acides.

**Revendications pour l'Etat contractant suivant : ES**

**1.** Procédé de préparation de composés de type 3,7-diazabicyclo[3.3.1]nonane, de formule générale I

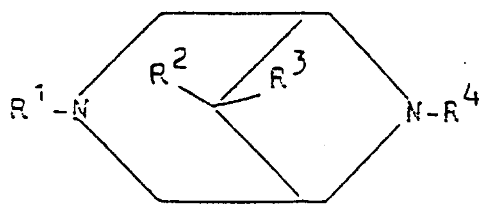

I

dans laquelle

| | |
|---|---|
| $R^1$ | représente un groupe alkyle ayant de 1 à 6 atomes de carbone, un groupe cycloalkylalkyle ayant de 4 à 9 atomes de carbone ou le groupe benzyle, |
| $R^2$ | représente un groupe alkyle ayant de 1 à 4 atomes de carbone et |
| $R^3$ | représente un groupe alkyle ayant de 1 à 4 atomes de carbone, ou |
| $R^2$ et $R^3$ | forment ensemble une chaîne alkylène ayant de 3 à 6 atomes de carbone, et |
| $R^4$ | représente un groupe cinnamyle de formule générale a |

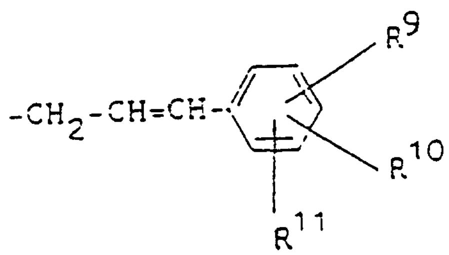

a

dans laquelle

| | |
|---|---|
| $R^9$ | représente un atome d'hydrogène ou d'halogène ou un groupe alkyle ayant de 1 à 4 atomes de carbone, alcoxy ayant de 1 à 4 atomes de carbone ou hydroxy, |
| $R^{10}$ | représente un atome d'hydrogène ou d'halogène ou un groupe alcoxy ayant de 1 à 4 atomes de carbone, alkyle ayant de 1 à 4 atomes de carbone, hydroxy, ou, lorsque $R^9$ est un atome d'hydrogène, également le groupe trifluorométhyle ou nitro, et |
| $R^{11}$ | représente un atome d'hydrogène ou, lorsque $R^9$ et $R^{10}$ sont des groupes alcoxy ayant de 1 à 4 atomes de carbone, représente également un groupe alcoxy ayant de 1 à 4 atomes de carbone, |

et de leurs sels d'addition avec des acides, caractérisé en ce que

a) on fait réagir des composés de formule générale II

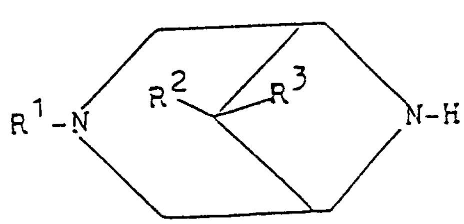

II

dans laquelle $R^1$, $R^2$ et $R^3$ ont les significations données plus haut, avec des composés de formule générale III

$R^4$-X III

dans laquelle $R^4$ a la signification donnée plus haut pour $R^4$, des groupes hydroxy éventuellement

libres étant cependant munis d'un groupe protecteur, et X représente un groupe séparable par aminolyse, ou

b) on réduit des composés de formule générale XIV

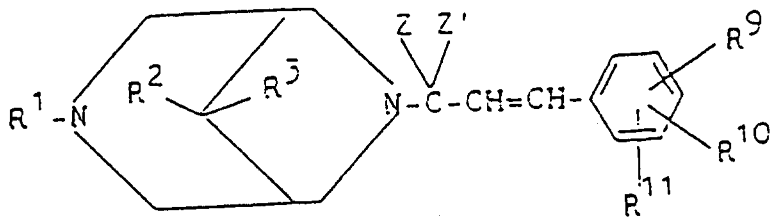

XIV

dans laquelle $R^1$, $R^2$, $R^3$ et $R^{11}$ ont les significations données plus haut, et $R^9$ et $R^{10}$ ont les significations données plus haut pour $R^9$ et $R^{10}$, des groupes hydroxy éventuellement libres étant toutefois munis d'un groupe protecteur, et Z et Z' représentant ensemble un atome d'oxygène, ou Z représente le groupe hydroxy et Z' représente un atome d'hydrogène, et on élimine ensuite les éventuels groupes protecteurs de fonction hydroxy et, si on le désire, on convertit des composés libres de formule I en leurs sels d'addition avec des acides ou les sels d'addition avec des acides en composés libres de formule I.

**2.** Procédé selon la revendication 1, caractérisé en ce que l'on prépare des composés dans lesquels $R^1$ représente un groupe alkyle ayant de 1 à 6 atomes de carbone ou un groupe cycloalkylalkyle ayant de 4 à 9 atomes de carbone.

**3.** Procédé selon la revendication 2, caractérisé en ce que l'on prépare des composés dans lesquels le groupe cycloalkylalkyle contient de 4 à 7 atomes de carbone.

**4.** Procédé selon la revendication 1, caractérisé en ce que l'on prépare des composés dans lesquels $R^{11}$ représente un atome d'hydrogène.

**5.** Procédé de préparation de composés de formule générale XIV'

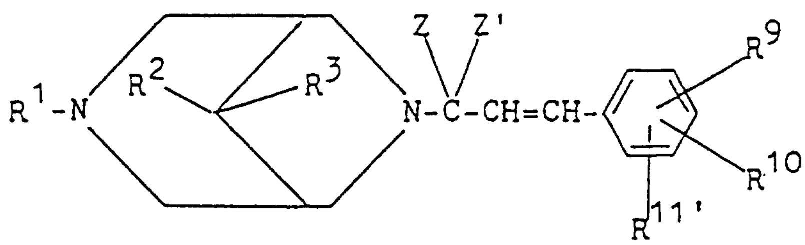

XIV'

dans laquelle $R^1$, $R^2$, $R^3$, $R^9$ et $R^{10}$ ont les significations données dans la revendication 1,

$R^{11'}$ représente un atome d'hydrogène, ou, lorsque $R^9$ et $R^{10}$ sont des groupes alcoxy ayant de 1 à 4 atomes de carbone et $R^2$ et $R^3$ sont des groupes alkyle ayant de 1 à 4 atomes de carbone, représente également un groupe alcoxy ayant de 1 à 4 atomes de carbone, et

Z et Z' représentent ensemble un atome d'oxygène, ou Z représente le groupe hydroxy et Z' représente un atome d'hydrogène,

et de leurs sels d'addition avec des acides, caractérisé en ce que

a) pour la préparation de composés de formule générale XIV'a

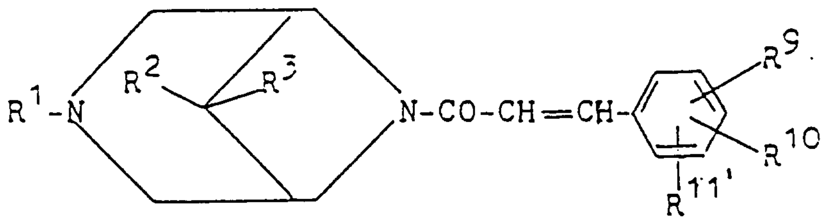

XIV'a

dans laquelle $R^1$, $R^2$, $R^3$, $R^9$, $R^{10}$ et $R^{11'}$ ont les significations données plus haut, on fait réagir des acides de formule générale XV'

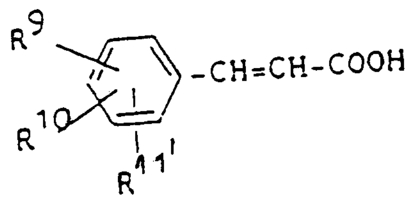

XV'

dans laquelle $R^9$, $R^{10}$ et $R^{11'}$ ont les significations données plus haut, ou leurs dérivés d'acides réactifs, avec des composés de formule générale II

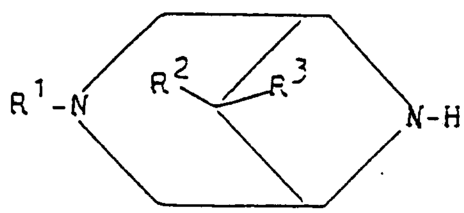

II

dans laquelle $R^1$, $R^2$ et $R^3$ ont les significations données plus haut, et

b) pour la préparation de composés de formule générale XIV'b

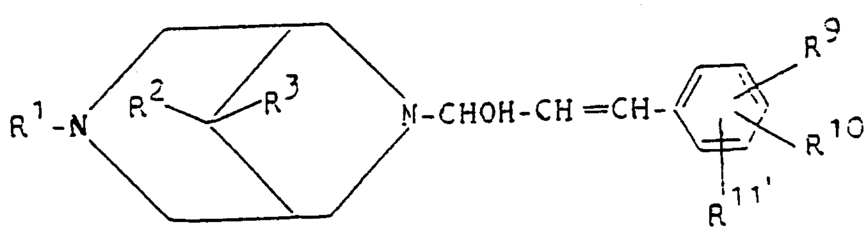

XIV'b

dans laquelle $R^1$, $R^2$, $R^3$, $R^9$, $R^{10}$ et $R^{11'}$ ont les significations données plus haut, on fait réagir des composés de formule II avec un aldéhyde de formule XVI'

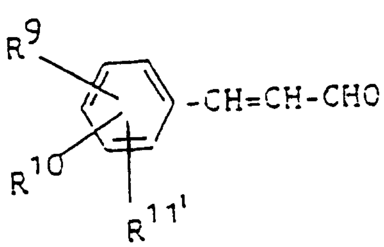

XVI'

dans laquelle $R^9$, $R^{10}$ et $R^{11}$ ont les significations données plus haut.